(19) Europäisches Patentamt
European Patent Office
Office européen des brevets

(11) **EP 4 284 496 B1**

(12) **EUROPÄISCHE PATENTSCHRIFT**

(45) Veröffentlichungstag und Bekanntmachung des Hinweises auf die Patenterteilung:
**22.04.2026 Patentblatt 2026/17**

(21) Anmeldenummer: **22709203.8**

(22) Anmeldetag: **01.02.2022**

(51) Internationale Patentklassifikation (IPC):
*A61N 1/378* (2006.01)   *H02J 50/90* (2016.01)
*A61N 1/362* (2006.01)

(52) Gemeinsame Patentklassifikation (CPC):
**A61N 1/3787; H02J 50/90;** A61N 1/362;
H02J 2105/46

(86) Internationale Anmeldenummer:
**PCT/EP2022/052313**

(87) Internationale Veröffentlichungsnummer:
**WO 2022/162238 (04.08.2022 Gazette 2022/31)**

(54) **LADEGERÄT**

CHARGER

CHARGEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.02.2021 DE 102021102244
16.04.2021 DE 102021109685
25.08.2021 EP 21193145**

(43) Veröffentlichungstag der Anmeldung:
**06.12.2023 Patentblatt 2023/49**

(73) Patentinhaber:
• **Mehnert, Walter**
  **85521 Ottobrunn (DE)**
• **Theil, Thomas**
  **82340 Feldafing (DE)**
• **Reimann, Rainer**
  **82386 Oberhausen (bei Peissenberg) (DE)**

(72) Erfinder:
• **Mehnert, Walter**
  **85521 Ottobrunn (DE)**
• **Theil, Thomas**
  **82340 Feldafing (DE)**
• **Reimann, Rainer**
  **82386 Oberhausen (bei Peissenberg) (DE)**

(74) Vertreter: **Kilian Kilian & Partner mbB
Zielstattstraße 23a
81379 München (DE)**

(56) Entgegenhaltungen:
WO-A1-2009/051539      WO-A1-2017/025606
WO-A1-2017/202455      DE-A1- 102011 078 883
US-A1- 2012 146 575      US-A1- 2018 229 045

**Beschreibung**

Die vorliegende Erfindung betrifft ein Ladegerät zur kontaktlosen Aufladung eines Energiespeichers eines Implantates.

Aus dem Patentdokument DE 10 2018 205 940 A1 ist ein elektronischer Schrittmacher, der vollständig in einen Körper eines Lebewesens zu implantieren ist, bekannt. Dieser Schrittmacher beinhaltet einen Energiespeicher, der eine Elektronik des Schrittmachers mit elektrischer Energie versorgt und der nach Entladung kontaktlos wieder aufgeladen werden kann.

Das kontaktlose Wiederaufladen des Energiespeichers erfolgt über "indirekte" Induktion unter Zuhilfenahme eines Magnetisierungsabschnittes, der auf ein magnetisches Wechselfeld derart anspricht, dass in ihm ab Erreichen einer bestimmten magnetischen Feldstärke eine Welle umklappender Domänen (weiß'sche Bezirke) auftritt. Das Auftreten der Welle ist unabhängig von der Frequenz des magnetischen Wechselfeldes, die lediglich Aufschluss darüber gibt, wie oft die Welle pro Zeiteinheit auftritt.

Diese Welle führt aufgrund der schnellen Änderung des magnetischen Flusses dazu, dass eine in räumlicher Nähe angeordnete Spule einen zur Aufladung des Energiespeichers führenden Spannungsimpuls abgibt.

Ein in dem Patentdokument gezeigtes Ladegerät erzeugt das magnetische Wechselfeld, wobei das Ladegerät während des Ladevorgangs auf oder in der Nähe einer Oberfläche des Körpers des Lebewesens angeordnet ist und das magnetische Wechselfeld den Magnetisierungsabschnitt des Schrittmachers in der erläuterten Weise triggern soll.

Das Ladegerät beinhaltet für die Erzeugung des magnetischen Wechselfeldes eine Vielzahl von Spulen. Der Bereich des magnetischen Wechselfeldes, der in die Oberfläche des Körpers eindringt und den Magnetisierungsabschnitt erreicht, ist Teil des schwachen Streufelds der Spulen.

Dieses Streufeld der Spulen ist zugleich äußerst inhomogen und nimmt mit zunehmender Entfernung von der Spule stark ab.

Aus diesem Grund erfordert das Wiederaufladen des Energiespeichers in Abhängigkeit von der Implantationstiefe des Schrittmachers eine minutiöse Einstellung der elektrischen Betriebsparameter der genannten Spulen und ein starkes Streufeld mit der entsprechenden Richtung des Feldvektors, damit die Welle umklappender Domänen im Magnetisierungsabschnitt zuverlässig und periodisch entsteht.

Weicht das Magnetfeld in dem Bereich des Magnetisierungsabschnitts zu stark von einem bestimmten optimalen Wert der Feldstärke bzw. Flussdichte ab, weil zu schwach oder zu inhomogen, unterbleibt die Auslösung der für die Aufladung notwendigen Welle.

Damit man die notwendige Feldstärke bzw. Flussdichte im Streufeld erreicht, muss das bekannte Ladegerät eine sehr hohe strom-/spannungsfeste Ausgestaltung haben.

Versuchte man den Energiespeicher mittels gewöhnlicher (frequenzabhängiger) Induktion nach dem Transformatorprinzip aufzuladen, sieht man sich neben den erläuterten Problematiken, insbesondere hinsichtlich Feldstärke/Flussdichte, noch größeren, sich negativ auswirkenden Effekten ausgesetzt.

Bei dieser Art der Aufladung wären an sich möglichst hohe Frequenzen wünschenswert, um eine adäquate Energieübertragung bei gleichzeitig annehmbarer Feldstärke/Flussdichte zu erreichen. Mit Erhöhung der Frequenz nehmen allerdings gleichzeitig gegenläufige Skineffekte im leitfähigen Gewebe des Körpers derart zu, dass das magnetische Wechselfeld in den Körper nur geringfügig eindringt und ein Erreichen des Implantates unmöglich ist.

Eine Erhöhung der Feldstärke/Flussdichte des Streufeldes am Ort des Implantates bei gleichzeitig niedriger Frequenz ist alternativ auch kaum möglich, weil induktive Wechselstromwiderstände von im Ladegerät sitzenden Spulen, so groß gewählt werden müssten, dass Strom- und/oder Spannungswerte in nicht mehr praktikablen Bereichen lägen.

Weiterer Stand der Technik findet sich in WO 2009/051539 A1, WO 2017/025606 A1 und US 2012/146575 A1.

Vor diesem Hintergrund ist es Aufgabe der Erfindung, ein Ladegerät zur kontaktlosen Aufladung eines Energiespeichers eines Implantates zu schaffen, das eine leichtere und zuverlässigere Erzeugung eines für die Aufladung notwendigen magnetischen Wechselfeldes zulässt.

Weiterhin ist es Aufgabe der Erfindung, ein zum Stand der Technik, insbesondere in Bezug auf Strom- und Spannungswerte, einfaches Ladegerät zu schaffen.

Diese Aufgabe(n) werden mit einem Ladegerät gemäß Patentanspruch 1 gelöst. Bevorzugte Ausgestaltungen des erfindungsgemäßen Ladegerätes sind Gegenstand der abhängigen Patentansprüche.

*Gemäß einem Aspekt der Erfindung beinhaltet das Ladegerät zur kontaktlosen Aufladung eines Energiespeichers eines Implantates, das in einen Körper eines Lebewesen implantiert ist, folgende Merkmale:*

*mindestens eine freistehende Spule, die sich entlang einer Spulenachse erstreckt und eingerichtet ist, ein magnetisches Wechselfeld zu erzeugen; wobei*

*der Körper bei bestimmungsgemäßer Benutzung des Ladegerätes derart räumlich relativ zu der Spule angeordnet ist, dass das magnetische Wechselfeld, das sich im Bereich innerhalb der Spule entlang der Spulenachse erstreckt, in den Körper zur Aufladung des Energiespeichers eindringt.*

Streng genommen erzeugt die Spule des Ladegerätes ein elektromagnetisches Wechselfeld. Für die Erfindung ist allerdings lediglich der magnetische Anteil des elektromagnetischen Wechselfelds von Belang. Deshalb spricht die vorliegende Beschreibung lediglich von einem magnetischen Wechselfeld.

Das Implantat, dessen Energiespeicher das erfindungsgemäße Ladegerät bestimmungsgemäß auflädt, ist beispielsweise ein Herzschrittmacher, ein Hirnschrittmacher, ein Organschrittmacher oder eine Analyseeinheit. Letztere Analyseeinheit ist beispielsweise so aufgebaut, dass sie kontinuierlich oder in bestimmten Zeitabständen Parameter, wie beispielsweise Blutdruck und/oder Blutwerte ermittelt. Besonders bevorzugt ist das Implantat ein Herzschrittmacher oder Herzschrittmachernetzwerk, der/das im menschlichen Herz oder am menschlichen Herz sitzt bzw. an diese Positionen implantiert ist.

Um unabhängig vom Wert der Frequenz des magnetischen Wechselfeldes zu werden, ist ein wesentliches Element des Implantats ein Magnetisierungsabschnitt mit ausgerichteten magnetischen Domänen, über den eine Ummagnetisierungswelle in Form der sich fortlaufend umpolenden magnetischen Domänen läuft, wenn die Amplitude (B-Feld) des von dem Ladegerät erzeugten magnetischen Wechselfeldes im Bereich des Magnetisierungsabschnittes einen bestimmten Wert erreicht. Der Magnetisierungsabschnitt ist beispielsweise ein Impulsdraht oder Wieganddraht.

Eine sich in räumlicher Nähe zu dem Magnetisierungsabschnitt befindende Spule erzeugt aufgrund der Ummagnetisierungswelle und der damit einhergehenden schnellen Änderung des magnetischen Flusses einen Spannungsimpuls, der beispielsweise eine Ladeelektronik mit Energie versorgt und zu einem Ladeimpuls führt. Mit dem Ladeimpuls kann der Energiespeicher geladen werden. Das von dem Ladegerät erzeugte magnetische Wechselfeld löst pro Umpolung die Ummagnetisierungswelle aus, wobei Ladeimpulse in der gleichen Anzahl auftreten und den Energiespeicher aufladen.

Alternativ kann das Implantat auch anders aufgebaut sein, dahingehend, dass sein Energiespeicher unter Ausnutzung gewöhnlicher, frequenzabhängiger Induktion aufgeladen wird. Hierfür besitzt das Implantat eine Empfangsspule mit einem bevorzugt speziell geformten Kern, wobei die Empfangsspule Ladeimpulse abgibt, die abhängig sind von der Frequenz und Betrag des erzeugten Wechselfeldes. Die Empfangsspule ist in Richtung einer Längserstreckung um den Kern gewickelt.

Das erfindungsgemäße Ladegerät dient folglich bestimmungsgemäß für ein Aufladen unter Ausnutzung "gewöhnlicher", frequenzabhängiger Induktion und/oder "indirekter", frequenzunabhängiger Induktion.

Ein Herzschrittmacher oder ein Herzschrittmachernetzwerk, die - die "indirekte" Induktion zur Aufladung nutzend - den im Vorhergehenden erläuterten Magnetisierungsabschnitt besitzen und deren Energiespeicher durch Erzeugung der Ummagnetisierungswelle aufgeladen werden, sind aus den Patentdokumenten DE 10 2019 124 435 und EP 3 756 726 A2 bekannt.

Die in diesen Patentdokumenten enthaltenen Ausführungen hinsichtlich der jeweiligen Ladeimpulserzeugungsabschnitte, insbesondere der Magnetisierungsabschnitte inklusive der um diese gewickelten und den jeweiligen Spannungsimpuls abgebenden Spulen, sowie hinsichtlich der jeweiligen Ladeelektronik werden durch Bezug aufgenommen.

Eine Frequenz f des von dem erfindungsgemäßen Ladegerät erzeugten magnetischen Wechselfeldes liegt insbesondere zwischen $0{,}1\,kHz \leq f \leq 10\,kHz$, insbesondere bei f= 2 kHz, 3 kHz, 4 kHz, oder 5 kHz. Die Frequenz f ist bevorzugt an die räumlichen Abmessungen und Materialdaten des Magnetisierungsabschnitts und eine damit in Zusammenhang stehende Laufzeit der über den Magnetisierungsabschnitt laufenden Ummagnetisierungswelle angepasst.

Die Frequenzen sind ebenfalls geeignet, um das Implantat in seiner alternativen Ausführung auf Basis gewöhnlicher Induktion aufzuladen, sofern die Empfangsspule des Implantates den speziell geformten Kern besitzt.

Die Form der freistehenden Spule des erfindungsgemäßen Ladegeräts kann unterschiedlich ausgestaltet sein. Beispielsweise kann die Spule kreisförmig, rechteckförmig oder quadratisch ausgebildet sein. Die Spule ist dahingehend ausgebildet, dass sie unabhängig von dem Körper des Lebewesens formstabil freistehend ist. D.h. der Körper kann sich relativ zu der formstabil stehenden Spule bewegen. Zudem hat die Spule solche räumlichen Abmessungen, dass der Körper des Lebewesens ohne jegliche Berührung mit der Spule oder deren Elemente, wie Rahmen oder Isolationen, relativ zu der Spule bewegt werden kann, sodass er sich letztendlich vollständig berührungslos zu der Spule und das Implantat sich in dem Bereich innerhalb der Spule bzw. in dem dortigen, sich entlang der Spulenachse erstreckenden magnetischen Wechselfeld befindet.

Die Spule des erfindungsgemäßen Ladegeräts erzeugt in dem Bereich, der sich innerhalb der Spule entlang der Spulenachse erstreckt, ein nahezu homogenes magnetisches Wechselfeld, wobei eine Amplitude der entsprechenden magnetischen Flussdichte in einem gesamten in Bezug auf die Spulenachse radialen Innenbereich der Spule solche Werte besitzt, dass

  i. es - unter der Annahme gleicher Ausrichtung zwischen dem magnetischen Feld (Richtung des B-Vektors) und bestimmungsgemäßer Laufrichtung der Ummagnetisierungswelle - pro Umpolung des magnetischen Wechselfeldes zur Auslösung der Ummagnetisierungswelle und damit zu dem Ladeimpuls kommt, oder

  ii. es unter der Annahme gleicher Ausrichtung zwischen dem magnetischen Feld (Richtung des B-Vektors) und bestimmungsgemäßer Ausrichtung des für die gewöhnliche Induktion dienenden Kerns der Empfangsspule - pro Umpolung des magnetischen Wechselfeldes zur Abgabe des Ladeimpulses kommt.

Aufgrund dieses Feldverlaufs kommt es auf die genaue Positionierung des Implantates in dem Bereich innerhalb der Spule nicht an, was eine minutiöse Einstellung des Wechselfeldes an die Gegebenheiten der Aufladung, wie beispielsweise Tiefe des Implantates, entbehrlich macht.

Bevorzugt ist das Ladegerät eingerichtet, einen Vektor des magnetischen Wechselfeldes zur Aufladung mindestens räumlich zweidimensional oder räumlich dreidimensional zu drehen. Diesbezüglich ist das Ladegerät bevorzugt eingerichtet, den Vektor des magnetischen Wechselfeldes zur Aufladung ohne Änderung der entsprechenden Amplitude des Vektors zu drehen. Die Drehung erfolgt mechanisch, wie es beispielsweise im Folgenden unter Bezug auf eine Aufhängung noch beschrieben wird, oder durch Veränderung sich überlagernder einzelner magnetischer Wechselfelder, die sich beispielsweise zwischen im Folgenden noch erläuterten Teilspulen befinden.

*Bevorzugt ist der Körper bei bestimmungsgemäßer Benutzung des Ladegerätes relativ zu der freistehenden Spule derart (berührungslos) angeordnet, dass eine Längsachse des Körpers in Richtung der Spulenachse verläuft und sich innerhalb der Spule befindet, wobei das magnetische Wechselfeld den Ort des Implantates erreicht.*

Bei dieser bevorzugten Ausgestaltung des Ladegeräts verläuft folglich die Körperlängsachse des Lebewesens, insbesondere des Menschen, in Richtung der Spulenachse, wobei diese Ausgestaltung insbesondere dann vorteilhaft ist, wenn die Ausrichtung der bestimmungsgemäßen Laufrichtung der über den Magnetisierungsabschnitt laufenden Ummagnetisierungswelle oder die Ausrichtung des für die gewöhnliche Induktion dienenden Kerns mit der Richtung der Körperlängsachse übereinstimmt. Dieser Zustand kann bevorzugt durch den Aufbau des Implantates so erreicht werden, dass die Form oder die Verankerungen des Implantates zu dieser Lage führt.

*Bevorzugt beinhaltet das erfindungsgemäße Ladegerät weiterhin:*
*eine Aufhängung, die die Spule haltert und eingerichtet ist, die Spule relativ zu dem Körper um mindestens eine, vorzugsweise zwei Achsen zur Drehung des Vektors zu schwenken, wobei das Ladegerät beispielsweise durch eine Steuerungseinheit bevorzugt eingerichtet ist, die Aufhängung anzusteuern, um die Spule zur Optimierung der Aufladung des Energiespeichers in eine bestimmte Ausrichtung relativ zu dem Körper zu schwenken.*

*Alternativ kann das Ladegerät bevorzugt eingerichtet sein, eine Anzeigevorrichtung anzusteuern, um einer Bedienperson anzuzeigen, in welche Richtung sie die Spule zur Optimierung der Aufladung des Energiespeichers relativ zu dem Körper manuell schwenken soll.*

Wenn die bestimmungsgemäße Laufrichtung der Ummagnetisierungswelle oder die Ausrichtung des für die gewöhnliche Induktion dienenden Kerns mit der Richtung des Vektors des magnetischen Wechselfeldes nicht übereinstimmt, kann es bei zu großen Abweichungen zu einem Absinken des Ladeimpulses kommen, weil der Anteil des Magnetfeldes in Richtung der Achse des Magnetisierungsabschnittes oder des Kerns zu klein wird.

Um eine Einstellung oder Anpassung der Laufrichtung der Ummagnetisierungswelle oder des Kerns an die Feldausrichtung bzw. vice versa zu ermöglichen, ist die Aufhängung vorgesehen. Die Aufhängung ist beispielsweise eine kardanische Aufhängung, die es der Steuerungseinheit des Ladegerätes ermöglicht, die Spule des Ladegeräts relativ zu dem Körper des Lebewesens um zwei Achsen zu schwenken, um die Anpassung der Ausrichtung (Vektor des B-Feldes) des von der Spule erzeugten magnetischen Wechselfeldes an die bestimmungsgemäße Laufrichtung der Ummagnetisierungswelle oder des Kerns zu erreichen. Alternativ kann die kardanische Aufhängung manuell verstellbar sein, wobei die Anzeigevorrichtung die entsprechende Richtung bevorzugt anzeigt.

Das Schwenken der Spule zieht vorteilhafter Weise keine Änderung der Amplitude der magnetischen Flussdichte nach sich, sondern ändert nur die Ausrichtung des magnetischen Wechselfeldes.

*Weiterhin bevorzugt ist die Aufhängung, die die freistehende Spule haltert, relativ zu dem Körper versetzbar und/oder eine Körperhalterung zur Halterung des Körpers ist relativ zu der Spule versetzbar, wobei das Ladegerät eingerichtet ist, die Aufhängung und/oder die Körperhalterung zu versetzen, um die freistehende Spule zur Optimierung der Aufladung des Energiespeichers in eine bestimmte Position relativ zu dem Körper zu bringen.*

Beispielsweise beinhaltet das Ladegerät eine lineare Führung, über die die gesamte Aufhängung der Spule, vorzugsweise in Richtung der Körperlängsachse, versetzbar ist. Das Versetzen der Aufhängung entlang der linearen Führung kann ein Anwender entweder manuell durchführen oder automatisch, indem er an einem Userinterface der Steuerungseinheit entsprechende Instruktionen gibt. Das Versetzen der freistehenden Spule (und/oder das erwähnte Schwenken) erfolgt so, dass der Körper des Lebewesens, insbesondere des Menschen, bis auf die Körperhalterung keine anderen Elemente des Ladegerätes, wie beispielsweise die Spule oder deren Elemente, berührt.

Das Ladegerät ist bevorzugt eingerichtet, zu überprüfen, ob oder inwieweit die Ausrichtung der Laufrichtung der Ummagnetisierungswelle oder des für die gewöhnliche Induktion dienenden Kerns mit der Ausrichtung des Magnetfeldes übereinstimmt. Beispielsweise ist das Ladegerät in der Lage, den Ladezustand des Energiespeichers in bestimmten zeitlichen Abständen kabellos abzufragen und einen Rückschluss auf den Wirkungsgrad der Aufladung anhand der Änderung des Ladezustandes des Energiespeichers, anhand der Umpolfrequenz und einer bekannten maximalen Höhe der Ladeimpulse zu ziehen.

*Bevorzugt ist allerdings, dass das Ladegerät eine Empfangseinheit aufweist, die eingerichtet ist, ein Qualitätssignal, das von dem Implantat ausgesendet wird und den Wirkungsgrad der Aufladung widerspiegelt, zu empfangen, und das Ladegerät ist eingerichtet, in Abhängigkeit von dem Qualitätssignal die Spule zur Optimierung der Aufladung in die bestimmte Ausrichtung zu schwenken und/oder in die bestimmte Position zu bringen.*

Das Qualitätssignal kann beispielsweise ein niederfrequentes Signal sein, das den menschlichen Körper und, wenn keine Antenne hierfür vorgesehen ist, die Hülle bzw. das Gehäuse des Herzschrittmachers durchdringt. Die Qualität des Ladeipulses verhält sich bevorzugt proportional zum Wert des Integrales ($\int i \, dt$). Bei guten Ladeimpulsen, d.h. mit sehr hoher Qualität beträgt der Wert beispielsweise bis zu 1000nC. Das die Qualität angebende Signal kann beispielsweise den Wert des Integrals des Stroms des Ladeimpulses über der Zeit ($\int i \, dt$), d.h. seinen Ladungsinhalt, angeben. Alternativ kann das Qualitätssignal folgendes Verhältnis angeben:

(Ladungsinhalt des abgegebenen Ladungsimpulses/maximal möglichem Ladungsinhalt).

Weiterhin alternativ kann das Qualitätssignal ein binäres Signal sein, das einen OK-Zustand einnimmt, wenn der Ladeimpuls einen bestimmten Schwellenwert übersteigt.

Die Steuerungseinheit steuert unter Beobachtung des Qualitätssignals die Aufhängung, beispielsweise die kardanische Aufhängung, und ermittelt aus verschiedenen Stellungen der Spule und den entsprechenden Qualitätssignalen eine optimale Stellung der Spule, in die die Steuerungseinheit die Spule abschließend schwenkt. In der erläuterten Alternative kann das Ladegerät auf der Anzeigevorrichtung Richtungen anzeigen, in die die Bedienperson die Spule schwenken soll.

Folgend sind bevorzugte Parameter des erfindungsgemäßen Ladegeräts und/oder seiner Spule:
*Die magnetische Flussdichte des magnetischen Wechselfeldes entlang der Spulenachse hat bevorzugt einen Wert B im mT-Bereich, bevorzugt größer/gleich 1mT, insbesondere in den folgenden Bereichen:*
1,0mT <= B <= 20,0mT, insbesondere *2,0mT <= B <= 20,0mT, 2,5mT <= B <= 8,0mT, 3,5mT <= B <= 7,0mT, 4,5mT <= B <= 6,0mT, 4,8mT <= B <= 5,2mT, oder 5,0mT = B.*

*Ein Durchmesser der Spule hat einen Wert D, der so bemessen ist, dass der Körper des Lebewesens, insbesondere des Menschen, ohne Berührung mit der Spule oder deren Elementen in das magnetische Wechselfeld, das sich im Bereich innerhalb der Spule entlang der Spulenachse erstreckt, bewegt werden kann und dort berührungslos relativ zu der Spule bestimmungsgemäß für die Aufladung angeordnet ist. Der Durchmesser liegt bevorzugt in folgenden Bereichen: 0,6m <= D <= 0,9m, 0,65m <= D <= 0,85m, 0,68m <= D <= 0,8m, oder 0,72m = D; und/oder eine Länge der Spule besitzt bevorzugt einen Wert l, wobei bevorzugt 0,15m <= l <= 0,5m, 0,2m <= l <= 0,45m, 0,25m <= l <= 0,4m, oder 0,33m = l ist.*

Die Spule des Ladegerätes kann eine kontinuierlich gewickelte Spule sein, insbesondere für den Fall, dass die Körperlängsachse des menschlichen Körpers mit der Spulenachse bestimmungsgemäß übereinstimmen soll.

Alternativ kann die Spule auseinandergezogen sein und aus zwei Teilspulen aufgebaut sein. Die Abmessungen der Teilspulen und deren Abstand zueinander sind bevorzugt so gewählt, dass die auseinandergezogene Spule wie eine kontinuierlich gewickelte Spule wirkt, zumindest in einem um die Spulenachse herum radialen Bereich. Ein Beispiel für eine auseinandergezogene Spule ist eine Helmholtzspule. Bestimmungsgemäß befindet sich der Körper des Menschen zwischen den Teilspulen und dem dortigen Magnetfeld bzw. magnetischen Wechselfeld, wobei die Körperlängsachse des Körpers senkrecht zur Spulenachse verläuft.

Die auseinandergezogene Spule kann auch durch eine verstellbare Aufhängung gehalten sein.

Weiterhin alternativ kann das Ladegerät zwei oder drei auseinandergezogene Spulen beinhalten, deren Spulenachsen bevorzugt in den zwei oder drei Raumkoordinaten angeordnet sind. Der menschliche Körper und das Implantat befinden sich bei bestimmungsgemäßer Benutzung des Ladegerätes zwischen den jeweiligen Teilspulen, wobei die von den auseinandergezogenen Spulen erzeugten magnetischen Wechselfelder, die sich jeweils in einem Bereich innerhalb der jeweiligen Spule befinden, sich zwischen den Teilspulen überlagern und das in den Körper implantierte Implantat erreichen.

Eine gezielte Ausrichtung des aus den überlagerten Wechselfeldern resultierenden magnetischen Wechselfelds kann durch unterschiedliche Ansteuerung der Phasen und/oder der Amplituden der jeweiligen Spulenpaare erreicht werden, um die Aufladung zu optimieren.

Die Ansteuerung des resultierenden Wechselfelds kann beispielsweise auf dem Qualitätssignal beruhen.

Wie aus der im Folgenden beschriebenen zweiten bevorzugten Ausführungsform und der dritten bevorzugten Ausführungsform des Ladegerätes ersichtlich ist, sind die im Vorhergehenden erwähnten, auseinandergezogenen Spulen freistehend, d.h. die jeweiligen im Abstand zueinander angeordneten Teilspulen sind formstabil und freistehend, sodass der Körper des Lebewesens (Menschen) in das magnetische Wechselfeld bewegt werden kann und dort für die Aufladung berührungslos zur Spule bzw. deren Elementen angeordnet verbleibt.

Im Folgenden werden bevorzugte Ausführungen des erfindungsgemä-ßen Ladegeräts unter Bezug auf die beigefügten Figuren erläutert.

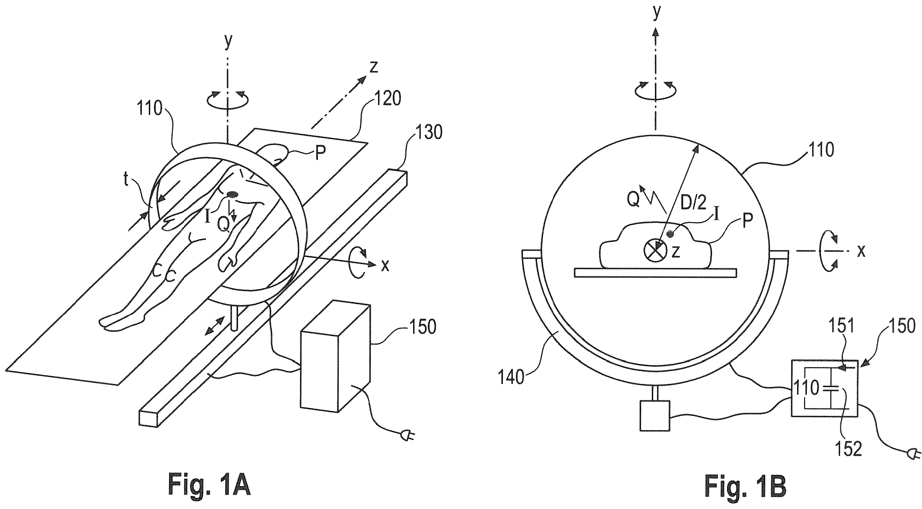

**Figuren 1A und 1B** zeigen eine erste bevorzugte Ausführungsform des erfindungsgemäßen Ladegeräts zum einen in einer perspektivischen Ansicht und zum anderen in einer Ansicht entlang einer in den Figuren gezeigten Z-Achse, wobei das Ladegerät eine Spule beinhaltet, die durch eine kardanische Aufhängung gehaltert wird.

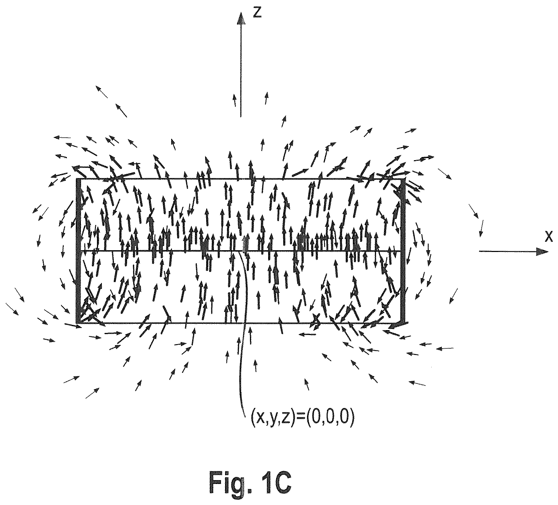

**Figur 1C** zeigt Simulationsergebnisse des magnetischen Wechselfeldes, das die Spule des Ladegerätes der ersten bevorzugten Ausführungsform der Erfindung erzeugt.

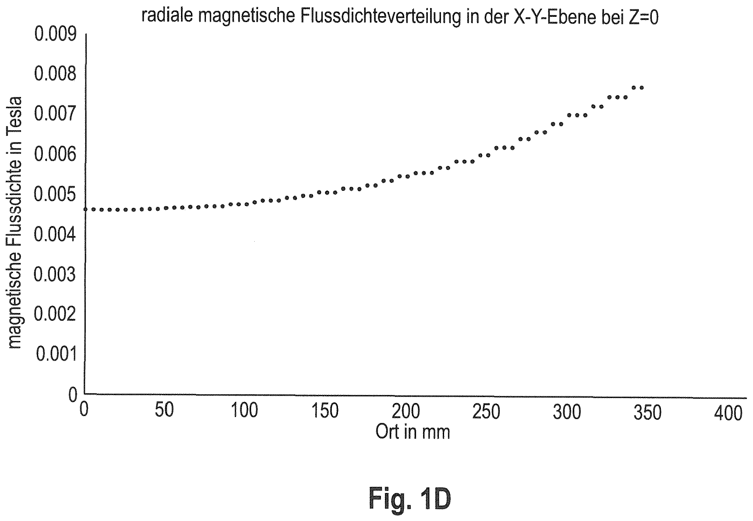

**Figur 1D** zeigt die Amplitude des magnetischen Wechselfeldes (B-Feld in mT) im Zentrum der Spule radial zur Spulenachse.

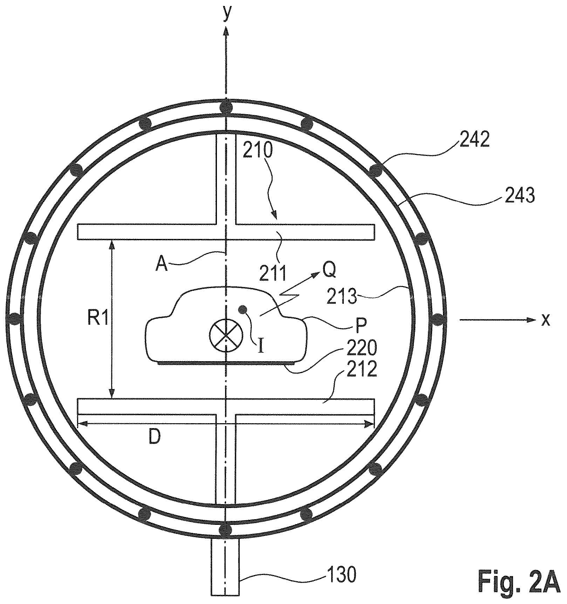

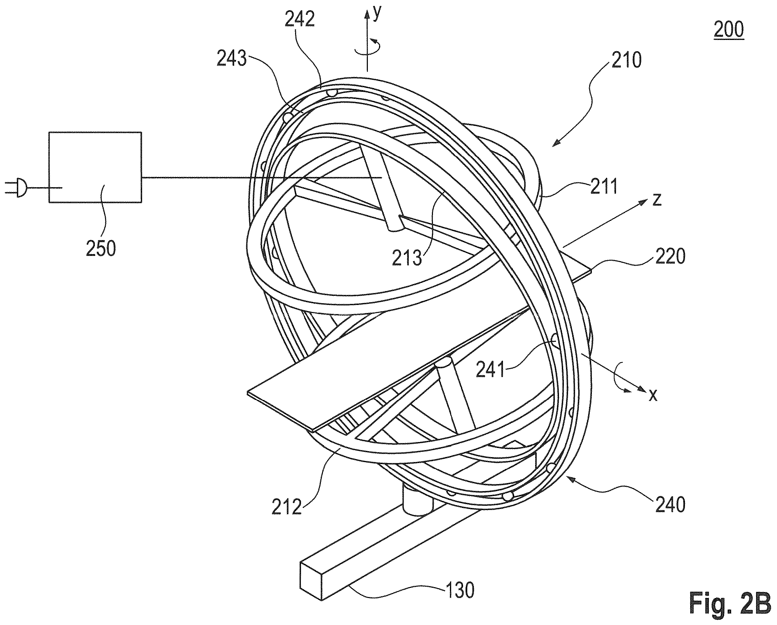

**Figur 2A und 2B** zeigen eine zweite bevorzugte Ausführungsform des erfindungsgemäßen Ladegeräts, welche eine Spule aus einem Spulenpaar beinhaltet, das aus zwei Teilspulen aufgebaut ist, wobei eine Aufhängung beide Teilspulen haltert und eingerichtet ist, die Teilspulen zusammen zu schwenken und/oder zu versetzen.

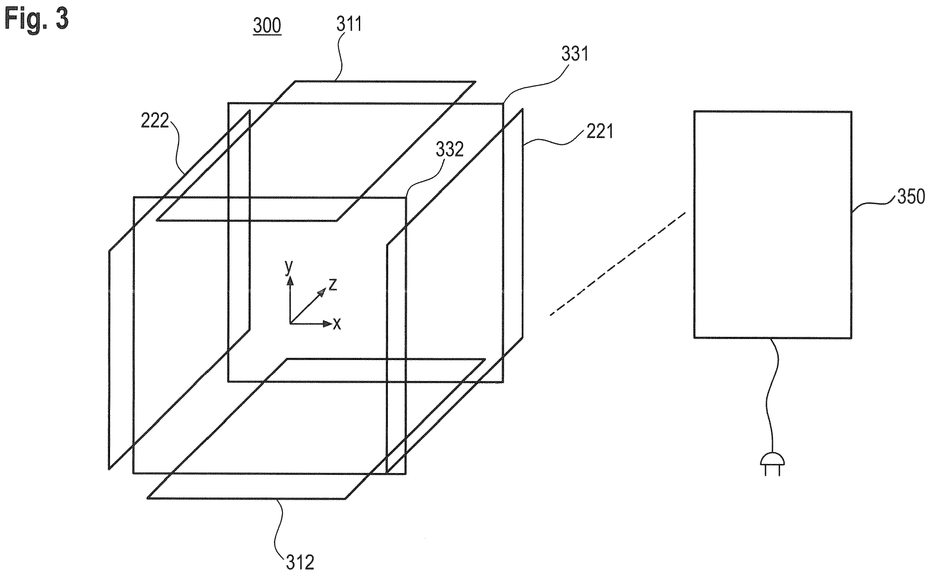

**Figur 3** zeigt eine dritte bevorzugte Ausführungsform des erfindungsgemäßen Ladegeräts, das drei Spulen beinhaltet, die jeweils aus zwei Teilspulen aufgebaut sind, wobei jeweilige Spulenachsen räumlich senkrecht aufeinander stehen und in einem Überlagerungsbereich, der sich zwischen den jeweiligen Spulen befindet, die Amplitude und der Vektor des magnetischen Wechselfelds durch Ansteuerung der Spulen einstellbar ist.

Die im Folgenden erläuterten Ausführungsformen des erfindungsgemäßen Ladegeräts dienen zum Aufladen eines Energiespeichers eines Implantates, das vollständig in einen menschlichen oder tierischen Körper implantiert ist.
Das Implantat ist allgemein eine Entität, die bestimmte Funktionen im implantierten Zustand übernimmt und zu diesem Zweck zumindest eine Elektronik und den genannten, die Elektronik versorgenden Energiespeicher aufweist.
Bevorzugt kann das Implantat noch Elektroden zur Erfassung von Körperdaten (Körperinformationen) und/oder Abgabe von Impulsen an den Körper sowie bevorzugt eine Kommunikationseinheit, die als Schnittstelle zur Außenwelt fungiert, aufweisen.
Das Implantat ist beispielsweise ein Herzschrittmacher, Gehirnschrittmacher, Blasenschrittmacher und/oder eine Analyseeinheit, die Körperdaten, wie Blutdruck und/oder Blutwerte, erfasst.
Für das kontaktlose Wiederaufladen des Energiespeichers besitzt das Implantat

i. einen Ladeimpulserzeugungsabschnitt mit einer Ladeelektronik, wie sie beispielsweise in dem Patentdokument DE 10 2019 124 435 beschrieben sind; und/oder
ii. eine Empfangsspule, die um einen bevorzugt speziell geformten Kern gewickelt ist, wobei die Empfangsspule mit Kern eine Aufladung des Energiespeichers auf Basis gewöhnlicher Induktion ermöglicht.

Wesentliches Element des Ladeimpulserzeugungsabschnitt ist ein Magnetisierungsabschnitt, beispielsweise ein Wieganddraht, mit ausgerichteten magnetischen Domänen, die durch die magnetische Feldkomponente in seiner Achsrichtung eines von den Ausführungsformen des erfindungsgemäßen Ladegeräts erzeugten elektromagnetischen Wechselfeldes dahingehend beeinflusst werden können, dass eine Ummagnetisierungswelle in Form von dominoartig umklappenden Domänen (weiß'sche Bezirke) über den Magnetisierungsabschnitt läuft.
Die Ummagnetisierungswelle führt zu einer so hohen zeitlichen Änderung des magnetischen Flusses, dass eine in einer in räumlicher Nähe zu dem Magnetisierungsabschnitt angeordnete Spule einen zu einem Ladeimpuls des Energiespeichers führenden Spannungsimpuls erzeugt.
Diese Art der Wiederaufladung des Energiespeichers ist insbesondere dahingehend vorteilhaft, als dass die Frequenz des magnetischen Wechselfeldes soweit abgesenkt werden kann, dass keine nachteiligen Effekte, wie Skin-Effekte, das magnetische Wechselfeld am Eindringen in den Körper und Erreichen des Magnetisierungsabschnitts hindern.
Beispielsweise kann der Magnetisierungsabschnitt ein Wieganddraht sein, um den eine Spule gewickelt ist.
Die Geschwindigkeit der über den Wieganddraht laufenden Ummagnetisierungswelle beträgt im Leerlauf größenordnungsmäßig 800 m/s, wobei die Länge des in dem Schrittmacher enthaltenen Wieganddrahtes in einem Bereich von 0,7 - 1,2cm liegt. Damit ergibt sich auch wieder im Leerlauf eine Laufdauer der Ummagnetisierungswelle (Welle umklappender Domänen) in der Größenordnung von 10-20 µs.
Unter Berücksichtigung dieser Werte erzeugt das im Folgenden noch erläuterte, erfindungsgemäße Ladegerät das magnetische Wechselfeld mit einer Frequenz in einem Bereich von 0,1 bis 10 kHz. Das magnetische Wechselfeld dringt in diesem Frequenzbereich in sehr tiefliegende Bereiche des Körpers des Lebewesens ein und kann damit problemlos den Magnetisierungsabschnitt des Implantats zur kontaktlosen Aufladung erreichen.
Der genannte Frequenzbereich eignet sich auch zur Aufladung mittels gewöhnlicher Induktion, zumindest wenn der bereits genannte Kern durch die Spule verläuft.
Die Ausführungsformen des erfindungsgemä-

ßen Ladegeräts werden im Folgenden unter der Annahme erläutert, dass es sich bei dem Implantat um einen Herzschrittmacher handelt, der vollständig in das/an das menschliche Herz implantiert ist. Beschränkt ist die Erfindung hierauf allerdings nicht.

Der Herzschrittmacher besitzt ein Gehäuse, das den Energiespeicher und die entsprechende Elektronik eingekapselt aufnimmt. Das Gehäuse des Herzschrittmachers besitzt ein Volumen in der Größenordnung von 1 cm$^3$.

Notwendige Elektroden des Herzschrittmachers liegen auf der Oberfläche des Gehäuses frei und berühren Abschnitte des menschlichen Herzen, und/oder sind als Verankerungselektroden ausgebildet, die in dem menschlichen Herzen verankert sind und den Herzschrittmacher ortsfest halten.

Der Herzschrittmacher erhält über die Elektroden Körperdaten, d. h. Informationen über die Aktivität des Herzens, und/oder kann hierüber Stimulationsimpulse an das Herz abgeben.

Der Energiespeicher des Herzschrittmachers ist beispielsweise ein elektrochemischer Energiespeicher, insbesondere ein Akkumulator, beispielsweise ein Lithium-Ionen-Akkumulator, mit einer solchen Kapazität, dass er alle elektronischen Komponenten des Herzschrittmachers für einen Zeitraum zwischen beispielsweise 0,75 bis 1,25 Jahren mit elektrischer Energie versorgen kann. Wird er beispielsweise alle 0,5 Jahre wieder aufgeladen, ist damit die Versorgung aller elektronischen Komponenten mit Energie sichergestellt.

Die Aufladung erfolgt kontaktlos auf Basis gewöhnlicher oder indirekter Induktion durch das im Folgenden noch näher beschriebene Ladegerät.

(Erste Ausführungsform)

Unter Bezug auf Figuren 1A bis 1E wird im Folgenden eine erste bevorzugte Ausführungsform des erfindungsgemäßen Ladegeräts erläutert.

Das Ladegerät 100 gemäß der ersten bevorzugten Ausführungsform beinhaltet eine freistehende (formstabile) Spule 110, die das für die Aufladung notwendige magnetische Wechselfeld erzeugt. Die Spule 110 besitzt solche räumliche Abmessungen, dass eine für einen Patienten P vorgesehene Körperhalterung 120 des Ladegerätes 100 abschnittsweise innerhalb der Spule 110 Platz findet, ohne mit der freistehenden Spule 110 oder deren Elementen, wie Isolationen, Rahmen, Wicklungen etc., in Berührung zu kommen.

Die Körperhalterung 120 ist in der bevorzugten Ausführungsform des Ladegeräts 100 eine Liege, auf der der Patient P bei bestimmungsgemäßer Benutzung des Ladegerätes 100 liegt bzw. angeordnet ist.

Der Patient P ist Träger eines schematisch angedeuteten Herzschrittmachers I, der vollständig in das Herz des Patienten P implantiert ist.

Die Körperhalterung 120 verläuft in Richtung einer in Figuren 1A und 1B gezeigten Z-Achse durch die Spule 110 hindurch. Die Z-Achse entspricht einer Spulenachse der Spule 110. Wenn der Patient P, wie es im Folgenden noch beschrieben wird, auf der Körperhalterung 120 liegt, verläuft eine Körperachse des Patienten in Richtung der Spulenachse der Spule 110, wobei das magnetische Wechselfeld, das sich in dem Bereich innerhalb der Spule 110 befindet, in den Körper des Patienten P eindringt und den Herzschrittmacher I zur Aufladung des Energiespeichers erreicht.

Die Spule 110 ist in der bevorzugten Ausführungsform eine kreisförmige Spule mit einem Durchmesser D, wobei eine entsprechende Spulenebene in der in Figuren 1A und 1B gezeigten X-Y-Ebene senkrecht zur Spulenachse (Z-Achse) liegt.

Alternativ kann die Spule 110 auch eine quadratische oder rechteckförmige Rahmenspule sein. Der Durchmesser D beträgt in der ersten bevorzugten Ausführungsform 0,72 m, kann allerdings insoweit variieren, solange der Patient berührungslos zur Spule in ihr Platz findet.

Die Spule 110 besitzt beispielsweise eine einschichtige Wicklung mit einer Vielzahl von Windungen w, die aus einer elektrischen Leitung, beispielsweise einer Kupferleitung, gebildet ist. In der ersten bevorzugten Ausführungsform beträgt die Anzahl der Windungen w=10, wobei dieser Wert lediglich bevorzugt ist und ebenfalls variieren kann.

Die die Wicklung bildende, elektrische Leitung besitzt bevorzugt einen rechteckigen Querschnitt von 320 mm$^2$, der sich aus einer in Richtung der Z-Achse weisenden Seitenlänge von 32 mm und eine hierzu senkrecht verlaufende Seitenlänge von 10 mm ergibt. Hinzu kommt eine elektrische Isolierung, die die Oberfläche der elektrischen Leitung überzieht, mit einer Stärke von 0,5 mm.

Damit resultiert eine Länge I der Spule 110 in Richtung ihrer Spulenachse (Z-Achse) von 0,32 m ohne Berücksichtigung der Isolierung bzw. von 0,33 m mit Berücksichtigung der Isolierung, wobei für die erste bevorzugte Ausführungsform damit die Bedingung D > I Geltung besitzt.

Eine Induktivität L der Spule 110 hat bei diesem Aufbau der Spule 110 einen Wert von 87 μH. Die Erfindung ist nicht auf den erläuterten Aufbau und die genannten Parameter eingeschränkt. Allgemein ist es bevorzugt, dass der Aufbau der Spule 110 so gestaltet ist, dass die Induktivität in dem folgenden Bereich liegt: 0,02mH <= L <= 0,3mH.

Die Spule 110 des erfindungsgemäßen Ladegeräts 100 wird bevorzugt während der Aufladung des Energiespeichers des Herzschrittmachers I so betrieben, dass sie in ihrem Innenraum das magnetische Wechselfeld mit einer Amplitude (magnetische Flussdichte B) im Bereich von größer/gleich 1mT, bevorzugt im Bereich von 2,5mT <= B <= 8,0mT, insbesondere in einem Bereich von 5mT, erzeugt.

Das magnetische Wechselfeld mit diesen Werten der magnetischen Flussdichte B befindet sich in dem Bereich innerhalb der Spule 110 und verläuft entlang der Spulenachse der Spule 110.

Der in dem Herzschrittmacher I enthaltene Magnetisierungsabschnitt, der bevorzugt der bereits genannte Wieganddraht ist, zeigt bei den genannten Werten der magnetischen Flussdichte B und einer Frequenz f (Umpolfrequenz) des magnetischen Wechselfeldes in dem bereits genannten Bereich von 0,1 bis 10 kHz, insbesondere bei 2 kHz, ein dahingehend zuverlässiges Verhalten, dass jede Umpolung des magnetischen Wechselfeldes mit sehr hoher Wahrscheinlichkeit die Ummagnetisierungswelle ausgelöst und damit zu dem Ladeimpuls des Energiespeichers führt.

Auch lässt sich der Energiespeicher durch gewöhnliche Induktion in diesem Frequenzbereich, insbesondere bei Frequenzen ab 2kHz, wenn die Empfangsspule des Herzschrittmachers den geeigneten Kern besitzt, laden.

Der erläuterte Aufbau des erfindungsgemäßen Ladegeräts 100 gemäß der ersten bevorzugten Ausführungsform ist insbesondere vorteilhaft, weil elektrische Betriebsparameter in Größenordnungen liegen, die den problemlosen Betrieb des Ladegeräts 100 in einer gewöhnlichen Arztpraxis ermöglichen.

Die magnetische Flussdichte B des von der Spule 110 erzeugten magnetischen Wechselfeldes ergibt sich aus folgender Beziehung:

$$B=(\mu_0*I*w)/D \qquad (1)$$

Das erfindungsgemäße Ladegerät 100 erzeugt bevorzugt in der Wicklung der Spule 110 einen elektrischen Wechselstrom mit einer Stärke von I = 300 A. Bei den genannten w=10 Windungen ergibt sich aus der vorstehenden Beziehung (1) eine magnetische Flussdichte B von 5,25mT, die für das zuverlässige Auslösen des Wieganddrahtes bzw. Magnetisierungsabschnitts oder für eine ausreichende gewöhnliche Induktion in den genannten Bereichen liegt.

Unter Berücksichtigung des Wechselstromwiderstands der Spule 110, der aus $\omega*L$, mit $\omega=2\pi f$, resultiert, folgt eine für die Erzeugung des Wechselstromes I notwendige Wechselspannung aus $U=I*\omega*L$ und beträgt damit bei einer Frequenz f von 2 kHz des magnetischen Wechselfeldes 327 V.

Sowohl der Wert des in der Wicklung erzeugten Wechselstromes I als auch der entsprechende Spannungswert U liegen in Größenordnungen, die in normalen Gebäuden/Arztpraxen zur Verfügung gestellt werden können. Das erfindungsgemäße Ladegerät 100 muss deshalb nicht zwingend in einer gesonderten Einrichtung, wie es beispielsweise für den Betrieb eines MRT der Fall ist, installiert werden.

Vorteilhaft wird dies dadurch realisiert, dass die Spule 110 des erfindungsgemäßen Ladegeräts 100 Teil eines Schwingkreises ist, beispielsweise eines Parallelschwingkreises. Auf diese Weise muss nur die Wirkleistung (Kupferverluste) aus dem Netz entnommen werden.

Figur 1B zeigt schematisch einen Verstärker 151 und einen Kondensator 152, die zusammen in einer Steuerungseinheit 150 des Ladegerätes 100 enthalten sind. Der Kondensator 152 bildet mit der Spule 110 zusammen den genannten Parallelschwingkreis.

Der Parallelschwingkreis ist so dimensioniert, dass er sich bei bestimmungsgemäßem Betrieb des Ladegeräts 100 in Resonanz befindet. Unter Berücksichtigung der Induktivität von L=87μH der Spule 110 ergibt sich aus der für den Resonanzfall geltenden Beziehung $1/(\omega*C)=\omega*L$ eine Kapazität des Kondensators zu C=72,8 μF.

Der ohmsche Widerstand R der Spule 110 ist im Wesentlichen bedingt durch das Material und die Abmessungen der die Wicklung bildenden elektrischen Leitung. In der vorliegenden ersten bevorzugten Ausführungsform des erfindungsgemäßen Ladegeräts 100 beträgt der ohmsche Widerstand R der elektrischen Leitung der Spule 110 ca. 1,24 mΩ.

Daraus resultiert eine Wirkleistungsaufnahme der Spule 110 im Beispiel bei entsprechendem Effektivwert $I_{eff}$ des durch die Spule 110 fließenden Wechselstromes zu 56 W. Diese Wirkleistungsaufnahme des Parallelschwingkreises kann eine in Gebäuden standardmäßig vorhandene Stromversorgung (bspw. 230 V; 50 Hz) problemlos liefern. D.h. das erfindungsgemäße Ladegerät 100 kann über die Steuerungseinheit 150 an eine Standardsteckdose angeschlossen werden. Figuren 1A und 1B zeigen ein solches Anschlusskabel schematisch.

Wenn der Energiespeicher des Herzschrittmachers I im implantierten Zustand wieder aufgeladen werden muss, legt sich der Patient P, wie es aus Figuren 1A und 1B ersichtlich ist, auf die Liege 120.

Eine bezüglich der Z-Richtung (positive oder negative Richtung der Z-Achse) räumliche Position des Patienten P bzw. der Liege 120 relativ zu der Spule 110 wird erreicht, indem die Steuerungseinheit 150 des Ladegeräts 100 eine im Folgenden noch erläuterte Aufhängung 140 der Spule 110 relativ zu der Liege 120/dem Patienten P versetzt. Die Aufhängung 140 haltert die formstabile Spule 110, so dass sie freistehend ist. Hierfür besitzt das Ladegerät 100 eine lineare Führung 130, die unterhalb der Liege 120 angeordnet ist und die die Aufhängung 140 der Spule 110 in Z-Richtung versetzbar haltert.

Die Steuerung 150 ist eingerichtet, die lineare Führung 130 anzusteuern um die Aufhängung 140 an eine bestimmte Position in Z-Richtung zu versetzen. Alternativ/zusätzlich kann die Körperhalterung/Liege 120 versetzbar gehalten sein, sodass die Steuerungseinheit 150 des Ladegeräts 100 durch Ansteuerung von Verstellungsmechanismen die Körperhalterung/Liege 120 zur berührungslosen Positionierung des Patienten P relativ zur Spule 110 versetzen kann.

Der Patient P und die Spule 110 haben bei bestimmungsgemäßer Aufladung eine solche räumliche Relation zueinander, dass sich der Herzschrittmacher I in dem Bereich entlang der Spulenachse innerhalb der Spule 110 befindet.

Dieser Zustand ist in Figuren 1A und 1B dahingehend gegeben, dass sich der Herzschrittmacher I in dem von der Spule 110 definierten Zylindervolumen V, das sich aus $(D/2)^2*\pi*l$ ergibt, befindet. Das in diesem Bereich entlang der Spulenachse verlaufende magnetische Wechselfeld dringt in den Körper des Patienten P ein und erreicht den Herzschrittmacher I.

Die magnetische Flussdichte B besitzt in dem gesamten Zylindervolumen für die Aufladung adäquate Werte, weshalb die konkrete Position des Herzschrittmachers I innerhalb des Zylindervolumens V eine untergeordnete Rolle spielt, solange die Ausrichtung des Magnetisierungsabschnittes oder des für die gewöhnliche Induktion dienenden Kerns stimmt. Dies wird aus den folgenden Erläuterungen zu den Figuren 1C bis 1E ersichtlich.

Figuren 1C und 1D zeigen Stärke und Ausrichtung der magnetischen Flussdichte B des von der Spule 110 erzeugten magnetischen Wechselfeldes, wenn der Wechselstrom von I = 300 A durch die Wicklung der Spule 110 fließt. Figur 1C entspricht einer Schnittansicht der Spule 110, wobei die Schnittebene der in Figuren 1A und 1B gezeigten Z-X-Ebene entspricht und den Mittelpunkt der Spule 110 enthält. Der Mittelpunkt der Spule 110 ist gleichzeitig der Ursprung des in Figur 1A und 1B gezeigten Koordinatensystems.

Figur 1D zeigt in diesem Zusammenhang die Amplitude der magnetischen Flussdichte B und Figur 1C die entsprechende Vektordarstellung, wobei die Figuren die im Raum entsprechend resultierenden Größen betreffen.

Die Figur 1D macht deutlich, dass die Amplitude der magnetischen Flussdichte B innerhalb der Spule 110, insbesondere im Bereich der mittleren Ebene (X-Y-Ebene, mit Z = 0), über den gesamten Durchmesser D hinweg eine Amplitude aufweist, die über 4,5 mT liegt (siehe Bereiche in Figur 1D). Hinzu kommt, was Figur 1C verdeutlicht, dass die Ausrichtung (Vektor) der magnetischen Flussdichte B im Bereich der mittleren Ebene im Wesentlichen homogen ist, d. h. die die Ausrichtung der magnetischen Flussdichte B angebenden Vektoren verlaufen parallel zur Spulenachse.

Figur 1D zeigt im Detail, wie sich die Amplitude der resultierenden magnetischen Flussdichte B in der mittleren Ebene der Spule 110 (Z = 0) ausgehend von der Spulenachse bzw. dem Ursprung des Koordinatensystems in radialer Richtung (X- und/oder Y-Richtung) verändert. Die magnetische Flussdichte B besitzt bei (X, Y, Z) = (0, 0, 0) eine resultierende magnetische Flussdichte von 4,5 mT und steigt in radialer Richtung bis auf ungefähr 8 mT an.

Bestimmungsgemäß wird das Ladegerät 100 der ersten bevorzugten Ausführungsform so betrieben, dass sich der Herzschrittmacher I bei Aufladung des entsprechenden Energiespeichers in dem von der Spule 110 definierten Zylindervolumen V befindet. Es ist allerdings besonders bevorzugt, dass der Patient P bei bestimmungsgemäßem Betrieb des erfindungsgemäßen Ladegeräts 100 relativ zu der Spule 110 so angeordnet ist, dass sich der Herzschrittmacher I an einem Punkt in der mittleren (X-/Y-)-Ebene der Spule 110 befindet.

Der Magnetisierungsabschnitt, insbesondere der Wieganddraht, oder der Kern des Herzschrittmachers I ist bei bestimmungsgemäßem Betrieb des Ladegerätes 100 so ausgerichtet, dass die bestimmungsgemäße Laufrichtung der Ummagnetisierungswelle bzw. eine Längserstreckung des Kerns mit der Richtung der Spulenachse/Z-Achse und damit mit der hauptsächlichen Komponente (Z-Komponente) der resultierenden magnetischen Flussdichte B übereinstimmt. Die bestimmungsgemäße Laufrichtung entspricht jener Richtung, in der die Ummagnetisierungswelle zur Erzielung optimaler Spannungsimpulse bzw. Ladeimpulse über den Magnetisierungsabschnitt laufen soll. Für den genannten Wieganddraht entspricht diese Laufrichtung in der Regel seiner Längsachse.

Die Abstimmung des Magnetisierungsabschnitts/Laufrichtung der Ummagnetisierungswelle oder Längserstreckung des für die gewöhnliche Induktion dienenden Kerns an die Richtung der Spulenachse wird dadurch erreicht, dass bei Implantation des Herzschrittmachers I bzw. des darin enthaltenen Magnetisierungsabschnitts auf die Ausrichtung geachtet wird. Beispielsweise besitzt der Herzschrittmacher I so ausgebildete Elektroden und oder Verankerungen, dass sich die Ausrichtung des Magnetisierungsabschnittes/Längserstreckung des für die gewöhnliche Induktion dienenden Kerns in Richtung der Körperachse (und damit in Z-Achse/Spulenachse) bei Implantation zwangsläufig ergibt.

Die Ausrichtung des Magnetisierungsabschnitts, insbesondere die Ausrichtung der Längserstreckung des Wieganddrahtes, oder der Längserstreckung des für die gewöhnliche Induktion dienenden Kerns in Richtung der Z-Achse/Spulenachse führt dazu, dass jede Umpolung des elektromagnetischen Wechselfeldes die Ummagnetisierungswelle/gewöhnliche Induktion zuverlässig auslöst, und es zu einem gewünschten Spannungsimpuls, der wiederum zu dem Ladeimpuls für den Energiespeicher führt, kommt.

Die Erläuterungen zu dem von der Spule 110 erzeugten magnetischen Wechselfeld lassen erkennen, dass die zuverlässige Auslösung der Ummagnetisierungswelle oder die zuverlässige Induktion unabhängig davon ist, wo sich der Herzschrittmacher bzw. der Magnetisierungsabschnitt in dem von der Spule 110 definierten Zylindervolumen V befindet. Dies deshalb, weil innerhalb der Spule 110 eine ausreichend homogene Amplitude der magnetischen Flussdichte B bei gleichzeitiger in Spulenachse weisende Ausrichtung des B-Vektors (ortsunabhängig) vorliegt.

Eine minutiöse Einstellung und Anpassung der Stärke und Ausrichtung des magnetischen Wechselfeldes an die Lage und Ort des Herzschrittmachers ist damit nicht erforderlich.

Auch wenn der Herzschrittmacher I in den Körper des Patienten P so implantiert worden ist, dass die Laufrichtung der Ummagnetisierungswelle oder die Längserstreckung des Kerns in Richtung der Körperachse des Patienten weist, kann es bei bestimmungsgemäßem Betrieb des Ladegeräts 100 zu Abweichungen zwischen Längserstreckung des Kerns oder der Ausrichtung des Magnetisierungsabschnittes und damit der Laufrichtung einerseits und der Spulenachse andererseits kommen.

Grund hierfür können zum einen individuelle physiologische Gegebenheiten des Patienten sein, die zu einer abweichenden Lage des Herzschrittmachers I in dem Körper des Patienten führen. Zum anderen können schlicht einfache körperliche Bewegungen des Patienten während des Ladevorgangs zu Abweichungen führen.

Um die Aufladung des Energiespeichers in einen optimalen Zustand zu bringen und/oder zu halten, ist die Aufhängung 140 verstellbar ausgebildet.

Zum einen kann die Spule 110 durch die lineare Führung 130 in Richtung der Z-Achse bzw. Spulenachse versetzt werden.

Zum anderen ist die Aufhängung 140 als kardanische Aufhängung ausgebildet. Das erlaubt ein Schwenken der Spule 110 um die jeweilige in Figuren 1A und 1B gezeigte X-Achse und Y-Achse.

Die Steuerungseinheit 150 ist eingerichtet, die genannten Einstellmöglichkeiten zur Optimierung der Aufladung des Energiespeichers zu nutzen.

Die Optimierung der Aufladung erfordert einen Rückschluss, inwieweit es durch Umpolung des magnetischen Wechselfeldes zu gewünschten, ausreichend hohen Ladeimpulsen kommt. Weicht nämlich die Ausrichtung der bestimmungsgemäßen Laufrichtung der Ummagnetisierungswelle oder der Längserstreckung des Kerns von der Spulenachse zu stark ab, vermindern sich die erzeugten Spannungsimpulse in ihrer Amplitude so weit, dass keine ausreichenden Ladeimpulse für den Energiespeicher mehr erreicht werden.

Der Herzschrittmacher I erzeugt bevorzugt als Rückmeldung ein Qualitätssignal Q, das den Wirkungsgrad der Aufladung widerspiegelt. Dieses Qualitätssignal kann aktiv gesendet oder passiv abgefragt werden.

Das gesendete Qualitätssignal Q kann beispielsweise ein niederfrequentes Signal sein, das den Köper des Pateinten P und, wenn keine externe Antenne hierfür vorgesehen ist, die Hülle bzw. das Gehäuse des Herzschrittmachers durchdringt.

Abgefragt werden kann das Qualitätssignal beispielsweise, indem der Herzschrittmacher definierte Frequenzen dämpft.

Die Qualität des Ladeipulses verhält sich proportional zum Wert des Integrales ($\int i\,dt$). Bei guten Ladeimpulsen, d.h. mit sehr hoher Qualität beträgt der Wert beispielsweise bis zu 1000nC. Das die Qualität angebende Signal Q kann beispielsweise den Wert des Integrals des Stroms des Ladeimpulses über der Zeit ($\int i\,dt$), d.h. seinen Ladungsinhalt, angeben.

Die Elektronik des Herzschrittmachers I kann diesbezüglich derart eingerichtet sein, dass sie das die Qualität angebende Signal Q für jeden Ladeimpuls aussendet oder alternativ nur für solche der Ladeimpulse die in bestimmten Intervallen hintereinander auftreten. Die Intervalle liegen beispielsweise bei 25, 50, 100, 200, 500, 750, oder 1000 Ladeimpulsen. Es besteht auch die Möglichkeit, einen Wert zu senden, der dem Mittelwert aller in einem Intervall aufgetreten Ladeimpulse entspricht. Auf diese Weise kann die für eine aktive Übertragung der Daten benötigte Energie reduziert werden.

Die Steuerungseinheit 150 besitzt eine Empfangseinheit und ist hierüber eingerichtet, dass Qualitätssignal Q von dem Herzschrittmacher I auszuwerten. Die Steuerungseinheit 150 führt auf der Basis des Qualitätssignals Q entsprechende Schritte aus um die Aufladung zu optimieren, indem sie die Spule 110 um die X-/Y-Achsen entsprechend schwenkt und/oder in Z-Richtung versetzt, bis das Qualitätssignal eine optimale Ausrichtung angibt.

Vorzugsweise verhält sich das Qualitätssignal Q proportional zum Wirkungsgrades der Aufladung.

Das die Qualität des Ladeimpulses angebende Signal Q kann alternativ beispielsweise ein binäres Signal sein, das einen OK-Zustand einnimmt, wenn der Ladeimpuls, bzw. sein Ladungsinhalt, einen Schwellenwert übersteigt, und einen NG-Zustand einnimmt, wenn der Ladeimpuls den Schwellenwert nicht übersteigt. Der Schwellenwert kann beispielsweise bei 50%, 55%, 60%, 65%, 70%, 75%, 80%, 85% oder 90% des durch den Magnetisierungsabschnitts lieferbaren Ladungsinhaltes liegen.

Wenn das Qualitätssignal Q ein binäres Signal ist, liegt der Schwellenwert für den OK-Zustand des Signals vorzugsweise in höheren Bereichen, weil der Schwellenwert insgesamt als Kriterium dafür dient, das Schwenken der Spule 110 um die jeweiligen Achsen zu beenden und die erreichte Stellung der Spule als optimal zu bewerten. Dazu werden beispielsweise die Extrempositionen (Wechsel von Gut auf Schlecht bzw. umgekehrt) ausgewertet.

Im Allgemeinen kann das erfindungsgemäße Ladegerät 100 auch bei Implantaten eingesetzt werden, die kein Qualitätssignal Q aussenden, sondern lediglich die Funktionalität besitzen, dass die Steuerungseinheit 150 den Ladezustand des Energiespeichers abfragen kann. Für den gezeigten Anwendungsfall kann beispielsweise die entsprechende Elektronik des Herzschrittmachers I so ausgestaltet sein, dass der Ladezustand des entsprechenden Energiespeichers über eine Sende- und Empfangsfunktion des Herzschrittmachers I abgefragt werden kann.

Damit kann die Steuerungseinheit 150 als Alternative zu dem die Qualität angebenden Signal Q den Rückschluss auf den Wirkungsgrad der Aufladung aus einer wiederholten Abfrage des Ladezustandes des Energiespeichers während der Aufladung gewinnen. Im Detail kann die Steuerungseinheit 150 durch Informationen über eine Änderung des Ladezustandes, den zeitlichen Abstand der Abfragen, einer bekannten maximalen Höhe der Ladeimpulse, und der Umpolfrequenz den Rückschluss auf die Qualität/den Wirkungsgrad der einzelnen Ladeimpulse ziehen. Der zeitliche Abstand, in dem die Steuerungseinheit 150 den Ladezustand des Energiespeichers abfragt, beträgt bevorzugt 0,5min, 1,0min, 1,5min, 2,0min, 2,5min, 3,0min, 3,5min, 4,0min, 4,5min, 5,0min.

Wenn die Steuerungseinheit 150 Erkenntnis über die Qualität der Ladeimpulse jeweils erlangt hat, steuert sie die kardanische Aufhängung 140 an, um die Spule 110 zur Optimierung der Aufladung um die jeweilige Achsen (X- Achse und/oder Y-Achse) zu schwenken und/oder entlang der Z-Achse zu versetzen. Das Schwenken und/oder Versetzen erfolgt so, dass der Patient auch hierbei mit der freistehenden (formstabilen) Spule oder deren Elementen nicht in Berührung kommt (berührungslos).

(Zweite Ausführungsform)

Im Folgenden wird eine zweite bevorzugte Ausführungsform der Erfindung unter Bezug auf Figuren 2A und 2B erläutert.

Figur 2A zeigt den Aufbau eines erfindungsgemäßen Ladegeräts 200 gemäß der zweiten bevorzugten Ausführungsform der Erfindung in Richtung der Z-Achse gesehen und Figur 2B eine perspektivische Ansicht.

Das Ladegerät 200 dient zur Aufladung eines Energiespeichers eines Herzschrittmachers I, der zu dem unter Bezug auf die erste Ausführungsform beschriebenen identisch aufgebaut ist. Insoweit wird hinsichtlich des Aufbaus und Funktionalität des Herzschrittmachers I auf die Ausführungen zur ersten Ausführungsform verwiesen.

Das Ladegerät 200 beinhaltet eine freistehende Spule 210, die eingerichtet ist, das für die indirekte Aufladung des Energiespeichers benötigte magnetische Wechselfeld zu erzeugen. Die Spulenachse A der Spule 210 verläuft in Figur 2A in Richtung der Y-Achse des dort gezeigten Koordinatensystems.

Die Wicklung der Spule 210 ist im Unterschied zu der ersten bevorzugten Ausführungsform nicht kontinuierlich, sondern bevorzugt in der Mitte der Spule 210, die gleichzeitig dem Ursprung des Koordinatensystems entspricht, derart auseinandergezogen, dass die Spule 210 aus Teilspulen aufgebaut ist.

Das Spule beinhaltet zwei kreisförmige Teilspulen 211 und 212, die axial zueinander angeordnet sind und den gleichen Durchmesser D besitzen. Die Teilspulen 211 und 212 sind in einem Abstand R1 zueinander angeordnet und weisen den gleichen Wicklungssinn auf.

Die Wicklung der Spule 210 verläuft über einen Verbindungsarm 213 und verbindet damit Teile der Wicklung, die die Teilspulen 211 und 212 bilden. Der Verbindungsarm 213 ist in dieser Ausführungsform ein geschlossener Ring, der bevorzugt kreisförmig ist.

Die Wicklung ist, wie in der ersten bevorzugten Ausführungsform einschichtig, und die die Teilspulen bildenden Abschnitte der Wicklung besitzen zwangsläufig denselben Wicklungssinn.

In der vorliegenden Ausführungsform hat die elektrische Leitung, die die Teilspulen 211, 212 bildet, bevorzugt dieselben Querschnittsabmessungen wie die in der ersten bevorzugten Ausführungsform; rechteckförmig mit 32 mm Seitenlänge in Richtung der Spulenachse und 10 mm Seitenlänge in zur Spulenachse radialer Richtung. Ein Abschnitt der elektrischen Leitung, der über den Verbindungsarm 213 läuft, kann die genannten Querschnittsabmessungen haben oder anders dimensioniert sein.

Jede der Teilspulen 211 und 212 hat beispielsweise einen Durchmesser von 160 cm und besitzt fünf Windungen (w = 5). Der Abstand R1 zwischen den Teilspulen 211 und 212 entspricht bevorzugt dem Radius der Teilspulen, d. h. in der vorliegenden Ausführungsform 80 cm, oder ist bevorzugt kleiner als der Radius. Der Durchmesser D der Teilspulen 211 und 212 kann größer gestaltet sein, wenn ein größerer Abstand R1 zwischen den Teilspulen benötigt wird.

Eine Aufhängung 240 haltert die formstabile Spule 210, so dass sie freistehend ist, indem sie den den Verbindungsarm 213 bildenden Ring 213 verstellbar lagert, wobei die Aufhängung 240 hierfür Verstellungsmechanismen aufweist.

Die Verstellungsmechanismen beinhalten eine Drehlagerung 241, die es erlaubt, den Verbindungsarm 213 und damit die Spule 210 in der in Fig. 2A und 2B gezeigten Stellung um die X-Achse des gezeigten Koordinatensystems zu schwenken bzw. zu drehen. Zudem besitzen die Verstellungsmechanismen Kugellager 242, die es ermöglichen, den Verbindungsarm/Ring 213, der über die Drehlagerung 241 mit einem Lagerring 243 verbunden ist, insgesamt um die in Figuren 2A und 2B gezeigte Z-Achse zu schwenken bzw. zu drehen. Wie hieraus verständlich wird, kann der Verbindungsarm/-Ring 213 durch die den Lagerring 243 lagernden Kugellager 242 so weit (90 Grad) gedreht werden, dass die Drehlagerung 241 nicht der in Figuren 2A und 2B gezeigten X-Achse, sondern der gezeigten Y-Achse entspricht.

Die Verstellungsmechanismen (Drehlager 241, Kugellager 242) ermöglichen, dass die Spule 210 in eine gewünschte Ausrichtung relativ zu dem implantierten Herzschrittmacher I ausgerichtet werden kann.

Zusätzlich kann das Ladegerät 200 der zweiten bevorzugten Ausführungsform der Erfindung die lineare Führung 130, die bereits unter Bezug auf die erste bevorzugte Ausführungsform erläutert wurde, beinhalten. Auf die entsprechenden Ausführungen im Rahmen der ersten bevorzugten Ausführungsform wird verwiesen. Wie auch in der ersten bevorzugten Ausführungsform kann die Liege 220 in Z-Richtung bevorzugt versetzbar sein. Wenn dies der Fall ist, ist die lineare Führung 130 bevorzugt nicht vorhanden.

Eine Steuerungseinheit 250, die eingerichtet ist, die unter Bezug auf die erste bevorzugte Ausführungsform erläuterten Funktionen auszuführen, ist sowohl mit der Aufhängung 240 als auch der Spule 210 elektrisch verbunden. Die Spule 210 ist, wie in der ersten bevorzugten Ausführungsform, Teil eines in Figur 2A nicht gezeigten Parallelschwingkreises, wobei die entsprechenden anderen Elemente des Schwingkreises, wie der bereits genannte Verstärker und Kondensator, in der Steuerungseinheit 250 aufgenommen sind.

Eine Körperhalterung 220, die wie in der ersten bevorzugten Ausführungsform eine Liege ist, verläuft zwischen den Teilspulen 211 und 212 senkrecht zur Zeichenebene der Figur 2A in Richtung der Z-Achse.

Wenn der Energiespeicher des Herzschrittmachers I des Patienten P aufgeladen werden muss, legt sich der Patient P auf die Liege 220 und befindet sich zwischen den Teilspulen 211 und 212. Die Körperachse bzw. Längsachse des Patienten P erstreckt sich wie die Liege 220 in Richtung der gezeigten Z-Achse. Der Patient P ist in Figur 2A schematisch gezeigt.

Die Spule 210 erzeugt ein einheitliches elektromagnetisches Wechselfeld, obwohl die Wicklung nicht kontinuierlich ist, sondern in Form der Teilspulen 211 und 212 auseinandergezogen ist. Andersrum ausgedrückt wirken beide Teilspulen 211, 212 aufgrund der im Vorhergehenden erläuterten Konfigurationen, Abmessungen und räumlicher Relation zueinander wie eine einheitliche Spule mit kontinuierlicher Wicklung. In der vorliegenden zweiten Ausführungsform besitzt die Spule 210 bevorzugt den Aufbau einer sog. Helmholtzspule.

Beide Teilspulen 211 und 212 erzeugen jeweils einen Teil des gesamt entstehenden magnetischen Wechselfeldes, wobei das magnetische Feld, das sich innerhalb der Spule 210 entlang der Spulenachse A erstreckt, Innenräume der Teilspulen 211, 212 und den Bereich zwischen den Teilspulen 211, 212 durchläuft.

Der Bereich zwischen den Teilspulen ist ein Überlagerungsbereich, in dem sich die von den Teilspulen erzeugten Teile des magnetischen Wechselfeldes so überlagern, dass das resultierende magnetische Wechselfeld sich entlang der Spulenachse A erstreckt.

Die die Ausrichtung angebenden Vektoren des magnetischen Wechselfelds, das die Innenräume der Teilspulen 211, 212 und den sich zwischen den Teilspulen befindenden Überlagerungsbereich durchläuft, zeigen parallele Verläufe. Zudem hat die magnetische Flussdichte B bei gleichen Annahmen/Betriebsparameter wie für die erste Ausführungsform gezeigt in dem Überlagerungsbereich Werte, die über weite Strecken über 4 mT liegen.

Diese Werte reichen aus, um die erläuterte Ummagnetisierungswelle, die über den Magnetisierungsabschnitt bzw. Wieganddraht des Herzschrittmachers I läuft und zu dem Ladeimpuls des Energiespeichers führt, oder die gewöhnliche zu dem Ladeimpuls führende gewöhnliche Induktion zu initiieren.

Im Gegensatz zu der ersten bevorzugten Ausführungsform durchdringt das magnetische Wechselfeld, das sich innerhalb der Spule entlang der Spulenachse A befindet, den Körper des Patienten P nicht parallel zu seiner in Z-Richtung verlaufenden Körperachse, sondern senkrecht hierzu.

Das Ladegerät 200 ist deshalb insbesondere für den Fall bestimmungsgemäß vorgesehen, dass der Herzschrittmacher I in dem Körper des Patienten P so ausgerichtet ist, dass die bestimmungsgemäße Laufrichtung der Ummagnetisierungswelle des Magnetisierungsabschnitts oder die Längserstreckung des Kerns nicht in Richtung der Körperachse (Z-Richtung) weist, sondern maßgeblich hiervon abweicht und im Wesentlichen in Y- und/oder X-Richtung verläuft.

Die Einstellung einer optimalen Position und Ausrichtung der Spule 210 in Bezug auf den Körper des Patienten P verläuft ähnlich wie bei der ersten bevorzugten Ausführungsform des erfindungsgemäßen Ladegeräts dadurch, dass die Steuerungseinheit 250 die Verstellungsmechanismen und/oder die lineare Führung 130 ansteuert und die Spule 210 relativ und berührungslos zu dem Patienten/Herzschrittmacher I solange verstellt und ausrichtet, bis eine optimale Aufladung stattfindet.

(Dritte Ausführungsform)

Figur 3 zeigt eine dritte bevorzugte Ausführungsform eines erfindungsgemäßen Ladegerätes 300. Das Ladegerät 300 dient ebenfalls zur Aufladung eines Energiespeichers eines Implantates I, wie beispielsweise der im Vorhergehenden erläuterte Herzschrittmacher I.

Bezüglich des Aufbaus und der Funktionalitäten des Herzschrittmachers I wird auf die Ausführungen zu der ersten und zweiten Ausführungsformen verwiesen.

Das Ladegerät 300 erzeugt das für die Aufladung des Energiespeichers notwendige magnetische Wechselfeld in einer ähnlichen Art und Weise wie die Spule 210 des Ladegeräts 200 gemäß der zweiten bevorzugten Ausführungsform, macht allerdings eine Aufhängung zur Änderung der räumlichen Ausrichtung des magnetischen Wechselfeldes entbehrlich.

Hierfür weist das Ladegerät 300 eine erste Spule, eine zweite Spule und eine dritte Spule auf, die zusammen freistehend und formstabil angeordnet sind.

Die erste Spule beinhaltet ein erstes Spulenpaar, das aus einer ersten Teilspule 311 und einer zweiten Teilspule 312 aufgebaut ist, wobei die entsprechende Spulenachse A in Richtung der in Figur 3 gezeigten Y-Achse des entsprechenden Koordinatensystems verläuft bzw. dieser entspricht.

Die zweite Spule beinhaltet ein zweites Spulenpaar, das gleichermaßen aus einer ersten Teilspule 221 und einer zweiten Teilspule 222 aufgebaut ist. Die Spulenachse B der zweiten Spule entspricht der in Figur 3 gezeigten X-Achse des entsprechenden Koordinatensystems.

Letztendlich beinhaltet das Ladegerät 300 eine dritte Spule, die wiederum aus einer ersten Teilspule 331 und einer zweiten Teilspule 332 aufgebaut ist. Die Spulenachse C der dritten Spule entspricht der Z-Achse des in Figur 3 gezeigten Koordinatensystems.

Alle Spulenachsen stehen bevorzugt räumlich senkrecht zueinander.

Die Spulen bzw. Teilspulen sind im Unterschied zu der vorangehenden Ausführungsformen nicht kreisförmig, sondern als Rahmenspulen mit rechteckförmigen Spulenebenen ausgebildet.

Alle Teilspulen, die jeweils die Spulenpaare der Spulen bilden, besitzen bevorzugt denselben Aufbau im Hinblick auf die die entsprechende Wicklung bildende elektrische Leitung und Windungsanzahl w und haben den jeweils gleichen Wicklungssinn. Abmessungen der elektrischen Leitung und Windungsanzahl w sind bevorzugt mit denen der ersten und zweiten bevorzugten Ausführungsform identisch. Die erste, zweite und dritte Spule sind bevorzugt so miteinander verbunden, dass sich gegenseitig stützen und zusammen formstabil, freistehend im Raum sind.

Ein Abstand zwischen den Teilspulen 221, 222 in X-Richtung ist im Verhältnis zu deren Abmessungen senkrecht zur Spulenachse A, d.h. in Ebenen parallel zu der in Figur 3 gezeigten Y-Z Ebene so dimensioniert, dass die Teilspulen 221, 222 wie eine einheitliche Spule (Helmholtzspule) wirken. Diese Bedingung gilt bevorzugt für alle in Figur 3 gezeigten Teilspulen.

Das Ladegerät 300 der dritten bevorzugten Ausführungsform beinhaltet eine Steuerungseinheit 350, die eingerichtet ist, jede Spule gesondert anzusteuern und einen Wechselstrom in den jeweiligen Wicklungen zu erzeugen. Jedes Spule bzw. die entsprechenden Teilspulen erzeugt damit ein individuelles magnetisches Wechselfeld.

Wie auch in den vorangehenden Ausführungsformen ist hierfür jede der Spulen Teil eines gesonderten Schwingkreises, deren Elemente in der Steuerungseinheit 350 aufgenommen sind. Bezüglich der Stärke des in jeder Spule erzeugten Wechselstroms und des Aufbaus der Schwingkreise wird auf die Ausführungen zu den vorangehenden Ausführungsformen verwiesen.

Wenn die Steuerungseinheit 350 den Wechselstrom in den Spulenpaaren/Teilspulen 311 und 312 erzeugt, verläuft das individuelle magnetische Wechselfeld, das sich innerhalb der Spule entlang der Spulenachse A befindet, durch die Teilspulen 311, 312 hindurch und über den sich zwischen den Teilspulen befindenden Überlagerungsbereich.

Die Teilspulen 311 und 312 erzeugen ergo das magnetische Wechselfeld in derselben Art und Weise wie die Spule 210 in der zweiten bevorzugten Ausführungsform. Diese Erläuterungen gelten für alle in Figur 3 gezeigten Teilspulen der entsprechenden Spulen.

Die individuellen magnetischen Wechselfelder aller Spulen überlagern sich in einem gemeinsamen Bereich zu dem magnetischen Wechselfeld, das das Implantat I erreicht.

Das magnetische Wechselfeld, das den Herzschrittmacher I erreicht, besitzt in dem gemeinsamen Bereich über weite räumliche Bereiche um den Koordinatenursprung herum ausreichend hohe und homogene hohe Werte, um den Magnetisierungsabschnitt (Wieganddraht) des Herzschrittmachers I zur Erzeugung des Ladeimpulses auszulösen oder den für die gewöhnliche Induktion dienenden Kern zur Erzeugung des Ladeimpulses zu durchströmen.

Die Ausrichtung des resultierenden magnetischen Wechselfeldes kann die Steuerungseinheit 350 dadurch verändern, dass sie die jeweiligen Spulen unterschiedlich ansteuert und den in den entsprechenden Wicklungen fließenden Wechselstrom variiert. Sofern die Ausrichtung des resultierenden magnetischen Wechselfeldes nicht im Raum, sondern nur in einer Ebene notwendig sein sollte, ist eine der drei Spulen entbehrlich.

Bei bestimmungsgemäßer Benutzung des Ladegeräts 300 wird der Patient P bevorzugt in Richtung der Z-Achse soweit durch die Teilspulen 331, 332 geschoben, dass sich der Oberkörper des Patienten P und der Herzschrittmacher I in dem gemeinsamen Bereich befindet. Auch bei dieser Ausführungsform erfolgt diese Bewegung des Patienten berührungslos zu allen Spulen bzw. deren Elementen.

Hierbei kann der Patient P wie auch in den anderen Ausführungsformen beispielsweise auf einer Körperhalterung bzw. Liege angeordnet sein.

Allen im Vorhergehenden erläuterten Ausführungsformen des erfindungsgemäßen Ladegerätes ist gemeinsam, dass das magnetische Wechselfeld, dass sich innerhalb der Spule(n) entlang der entsprechenden Spulenachse befindet, zur Aufladung des Energiespeichers benutzt wird. Das magnetische Wechselfeld bzw. dessen magnetische Flussdichte B ist in diesen Bereichen der Spule(n) über weite Strecken so stark und weitgehend homogen, dass eine genaue Kenntnis der Position des Implantats (Herzschrittmacher) von untergeordneter Bedeutung ist, wenn die Richtung der Achse des Magnetisierungsabschnittes oder der Längserstreckung des Kerns mit der Achse des Magnetfeldvektors in erster Näherung übereinstimmt.

Zudem lassen alle Ausführungsformen ein Schwenken/Drehen des Vektors des magnetischen Wechselfeldes zu, ohne dass sich die Amplitude der magnetischen Flussdichte B ändert. Dies erfolgt durch mechanisches Schwenken/Drehen der Spule(n) und/oder durch Veränderung der sich überlagernden magnetischen Wechselfelder ohne Berührung mit dem Patienten. Dies erlaubt eine überaus zuverlässige Ausrichtung des Magnetfeldvektors in Richtung der Längsachse des Kerns oder der Achse des Magnetisierungsabschnittes und damit in Laufrichtung der Ummagnetisierungswelle.

## Patentansprüche

1. Ladegerät (100, 200, 300) zur kontaktlosen Aufladung eines Energiespeichers eines Implantates (I), das in einen Körper eines Lebewesens implantiert ist, aufweisend:

   mindestens eine freistehende Spule (110, 210), die sich entlang einer Spulenachse erstreckt und eingerichtet ist, ein magnetisches Wechselfeld zu erzeugen; wobei
   der Körper bei bestimmungsgemäßer Benutzung des Ladegerätes (100, 200, 300) derart räumlich relativ zu der Spule (110, 210) angeordnet ist, dass das magnetische Wechselfeld, das sich im Bereich innerhalb der Spule entlang der Spulenachse erstreckt, in den Körper zur Aufladung des Energiespeichers eindringt.

2. Ladegerät (100, 200, 300) gemäß Patentanspruch 1, wobei das Ladegerät eingerichtet ist, einen Vektor des magnetischen Wechselfeldes zur Aufladung mindestens räumlich zweidimensional zu drehen.

3. Ladegerät (100, 200, 300) gemäß Patentanspruch 1 oder 2, wobei das Ladegerät eingerichtet ist, den Vektor des magnetischen Wechselfeldes zur Aufladung ohne Änderung der entsprechenden Amplitude zu drehen.

4. Ladegerät (100, 200, 300) gemäß Patentanspruch 1, 2 oder 3, wobei
   der Körper bei bestimmungsgemäßer Benutzung des Ladegerätes relativ zu der freistehenden Spule derart angeordnet ist, dass eine Längsachse des Körpers in Richtung der Spulenachse verläuft und sich innerhalb der Spule befindet, wobei das magnetische Wechselfeld den Ort des Implantates erreicht.

5. Ladegerät (100, 200, 300) gemäß Patentanspruch 2, 3 oder 4, weiterhin aufweisend:

   eine Aufhängung, die die freistehende Spule haltert und eingerichtet ist, die Spule relativ zu dem Körper um mindestens eine, vorzugsweise zwei Achsen zur Drehung des Vektors zu schwenken, wobei
   das Ladegerät eingerichtet ist, die Aufhängung anzusteuern, um die Spule zur Optimierung der Aufladung des Energiespeichers in eine bestimmte Ausrichtung relativ zu dem Körper zu schwenken.

6. Ladegerät (100, 200, 300) gemäß Patentanspruch 5, wobei

die Aufhängung, die die Spule haltert, relativ zu dem Körper versetzbar ist und/oder eine Körperhalterung zur Halterung des Körpers relativ zu der Spule versetzbar ist, und wobei
das Ladegerät eingerichtet ist, die Aufhängung und/oder die Körperhalterung zu versetzen, um die Spule zur Optimierung der Aufladung des Energiespeichers in eine bestimmte Position relativ zu dem Körper zu bringen.

7. Ladegerät (100, 200, 300) gemäß Patentanspruch 5 oder 6, wobei

   das Ladegerät eine Empfangseinheit aufweist, die eingerichtet ist, ein Qualitätssignal, das im Implantat erzeugt wird und den Wirkungsgrad der Aufladung widerspiegelt, entweder zu empfangen oder abzufragen, und
   das Ladegerät eingerichtet ist, in Abhängigkeit von dem Qualitätssignal die Spule zur Optimierung der Aufladung in die bestimmte Ausrichtung zu schwenken und/oder in die bestimmte Position zu bringen.

8. Ladegerät (100, 200, 300) gemäß einem der vorangehenden Patentansprüche, wobei
   eine magnetische Flussdichte des magnetischen Wechselfeldes entlang der Spulenachse einen Wert B aufweist, wobei

$$1,0mT <= B <= 20,0mT,$$

vorzugsweise

$$2,0mT <= B <= 20,0mT$$

$$2,5mT <= B <= 8,0mT,$$

$$3,5mT <= B <= 7,0mT,$$

$$4,5mT <= B <= 6,0mT,$$

$$4,8mT <= B <= 5,2mT,$$

oder

$$5,0mT = B$$

ist.

9. Ladegerät (100, 200, 300) gemäß einem der Patentansprüche 1, 2 oder 3, wobei die Spule aus zwei um einen Abstand R1 auf der gleichen Achse auseinander gezogenen Teilspulen aufgebaut ist, die derart zusammenwirken, dass das magnetische Wechselfeld entlang der Spulenachse die Teilspulen und einen sich zwischen den Teilspulen befindenden Bereich jeweils durchläuft; wobei vorzugsweise R1 gleich D/2 ist (Helmholtzspule) und der Körper bei bestimmungsgemäßer Benutzung des Ladegerätes derart relativ zu den Teilspulen so angeordnet ist, dass das, vorzugsweise vorwiegend homogene, magnetische Wechselfeld, das sich zwischen den Teilspulen befindet, in den Körper zur Aufladung des Energiespeichers eindringt.

10. Ladegerät (100, 200, 300) gemäß Patentanspruch 9, weiterhin aufweisend:

eine Aufhängung, die die zwei Teilspulen der ersten Spule haltert und eingerichtet ist, die erste Spule relativ zu dem Körper um mindestens eine, vorzugsweise zwei Achsen, zur Drehung des Vektors zu schwenken, und wobei das Ladegerät eingerichtet ist, die Aufhängung anzusteuern, um die zwei Teilspulen der ersten Spule zur Optimierung der Aufladung des Energiespeichers in eine bestimmte Ausrichtung relativ zu dem Körper zu schwenken.

11. Ladegerät (100, 200, 300) gemäß Patentanspruch 9, wobei das Ladegerät eingerichtet ist, die zwei Teilspulen der ersten Spule zur Optimierung der Aufladung des Energiespeichers in Abhängigkeit von einem Qualitätssignal, das von dem Implantat ausgesendet wird und den Wirkungsgrad der Aufladung widerspiegelt, in die bestimmte Ausrichtung relativ zu dem Körper zu schwenken.

12. Ladegerät (100, 200, 300) gemäß Patentanspruch 8 oder 9, weiterhin aufweisend:

eine zweite Spule, die sich entlang einer Spulenachse erstreckt und die aus zwei Teilspulen aufgebaut ist, die in einem Abstand R2 derart zueinander angeordnet sind, dass sich ein Bereich zwischen den Teilspulen der zweiten Spule befindet, wobei die Spulenachsen der ersten und zweiten Spule quer, bevorzugt senkrecht, zueinander verlaufen, derart, dass sich die zwischen den Teilspulen befindenden Magnetfelder der ersten und zweiten Spule in einem gemeinsamen Bereich überlagern und das Ladegerät eingerichtet ist, die erste Spule und die zweite Spule zur Optimierung der Aufladung des Energiespeichers derart anzusteuern, dass sich eine Richtung des Vektors des magnetischen Wechselfeldes in dem Überlagerungsbereich zweidimensional (in einer Ebene) dreht.

13. Ladegerät (100, 200, 300) gemäß Patentanspruch 12, weiterhin aufweisend:

eine dritte Spule, die sich entlang einer Spulenachse erstreckt und die aus zwei Teilspulen aufgebaut ist, die in einem Abstand R3 derart zueinander angeordnet sind, dass sich ein Bereich zwischen den Teilspulen der dritten Spule befindet, wobei die Spulenachsen der ersten, zweiten und dritten Spule quer, bevorzugt entlang von Raumkoordinaten X, Y und Z zueinander verlaufen, derart, dass sich die zwischen den Teilspulen befindenden Magnetfelder der ersten, zweiten und dritten Spule in dem gemeinsamen Bereich überlagern und das Ladegerät eingerichtet ist, die erste Spule, die zweite Spule und die dritte Spule zur Optimierung der Aufladung des Energiespeichers derart anzusteuern, dass sich die Richtung des Vektors des magnetischen Wechselfeldes in dem Überlagerungsbereich dreidimensional dreht.

14. Ladegerät (100, 200, 300) gemäß Patentanspruch 11 oder 12, wobei

das Ladegerät eingerichtet ist, ein Qualitätssignal, das im Implantat erzeugt wird und den Wirkungsgrad der Aufladung widerspiegelt, entweder zu empfangen oder abzufragen, und das Ladegerät eingerichtet ist, zur Optimierung der Aufladung des Energiespeichers den Vektor des magnetischen Wechselfeldes in dem Überlagerungsbereich in eine definierte Position zu bringen.

## Claims

1. Charging device (100, 200, 300) for contactless charging of an energy storage device of an implant (I) implanted in a body of a living being, comprising:

at least one free-standing coil (110, 210) extending along a coil axis and configured to generate an alternating magnetic field; wherein the body, during intended use of the charging device (100, 200, 300), is spatially arranged relative to the coil (110, 210) in such a manner that the alternating magnetic field, which extends within a region inside the coil along the coil axis, penetrates into the body for charging the energy storage device.

2. Charging device (100, 200, 300) according to claim 1, wherein the charging device is configured to rotate a vector of the alternating magnetic field at least spatially two-dimensionally for charging.

3. Charging device (100, 200, 300) according to claim 1 or 2, wherein the charging device is configured to rotate the vector of the alternating magnetic field for charging without changing the corresponding amplitude.

4. Charging device (100, 200, 300) according to claim 1, 2 or 3, wherein the body, during intended use of the charging device, is arranged relative to the free-standing coil in such a manner that a longitudinal axis of the body extends in the direction of the coil axis and is located within the coil, wherein the alternating magnetic field reaches the location of the implant.

5. Charging device (100, 200, 300) according to claim 2, 3 or 4, further comprising:

a suspension configured to hold the free-standing coil and configured to pivot the coil relative to the body about at least one axis, preferably two axes, for rotating the vector, wherein the charging device is configured to control the suspension to pivot the coil into a specific orientation relative to the body for optimizing charging of the energy storage device.

6. Charging device (100, 200, 300) according to claim 5, wherein

the suspension which supports the coil is displaceable relative to the body and/or a body support for supporting the body is displaceable relative to the coil, and wherein the charging device is configured to displace the suspension and/or the body support in order to bring the coil, for optimizing the charging of the energy storage, into a certain position relative to the body.

7. Charging device (100, 200, 300) according to claim 5 or 6, wherein

the charging device comprises a receiver unit configured to either receive or interrogate a quality signal generated in the implant and reflecting the efficiency of charging, and the charging device is configured, depending on the quality signal, to pivot the coil, for optimizing the charging, into the certain orientation and/or bring it into the certain position.

8. Charging device (100, 200, 300) according to one of the previous claims, wherein a magnetic flux density of the alternating magnetic field along the coil axis comprises a value B, wherein

$$1.0mT <= B <= 20.0mT,$$

preferably

$$2.0mT <= B <= 20.0mT,$$

$$2.5mT <= B <= 8.0mT,$$

$$3.5mT <= B <= 7.0mT,$$

$$4.5mT <= B <= 6.0mT,$$

$$4.8mT <= B <= 5.2mT,$$

or

$$5.0mT = B.$$

9. Charging device (100, 200, 300) according to one of the claims 1, 2, or 3, wherein the coil is composed of two partial coils spaced apart from one another by a distance R1 along the same axis, which cooperate in such a manner that the alternating magnetic field extends along the coil axis through the partial coils and through a region located between the partial coils; wherein preferably R1 is equal to D/2 (Helmholtz coil) and the body, during intended use of the charging device, is arranged relative to the partial coils in such a manner that the preferably predominantly homogeneous alternating magnetic field, which is located between the partial coils, penetrates into the body for charging the energy storage device.

10. Charging device (100, 200, 300) according to claim 9, further comprising:

a suspension configured to hold the two partial coils of the first coil and configured to pivot the first coil relative to the body about at least one axis, preferably two axes, for rotating the vector, wherein the charging device is configured to control the suspension in order to pivot the two partial coils of the first coil into a specific orientation relative to the body for optimizing charging of the energy storage device.

11. Charging device (100, 200, 300) according to claim 9, wherein the charging device is configured to pivot the two partial coils of the first coil, for optimizing charging of the energy storage device, into a specific orientation relative to the body as a function of a quality signal emitted by the implant and reflecting the efficiency of the charging.

**12.** Charging device (100, 200, 300) according to claim 8 or 9, further comprising:

a second coil extending along a coil axis and composed of two partial coils arranged at a distance R2 from one another in such a manner that a region is formed between the partial coils of the second coil, wherein
the coil axes of the first and second coils extend transversely, preferably perpendicularly, to one another, such that the magnetic fields located between the partial coils of the first and second coils overlap in a common region, and
the charging device is configured to control the first coil and the second coil, for optimizing charging of the energy storage device, in such a manner that a direction of the vector of the alternating magnetic field in the overlap region rotates two-dimensionally (in a plane).

**13.** Charging device (100, 200, 300) according to claim 12, further comprising:

a third coil extending along a coil axis and composed of two partial coils arranged at a distance R3 from one another in such a manner that a region is formed between the partial coils of the third coil, wherein
the coil axes of the first, second, and third coils extend transversely to one another, preferably along spatial coordinates X, Y, and Z, such that the magnetic fields located between the partial coils of the first, second, and third coils overlap in a common region, and
the charging device is configured to control the first coil, the second coil, and the third coil, for optimizing charging of the energy storage device, in such a manner that the direction of the vector of the alternating magnetic field in the overlap region rotates three-dimensionally.

**14.** Charging device (100, 200, 300) according to claim 11 or 12, wherein

the charging device is configured to either receive or interrogate a quality signal generated in the implant reflecting the efficiency of charging, and
the charging device being configured to bring the vector of the alternating magnetic field in the overlap region into a defined position.

**Revendications**

**1.** Chargeur (100, 200, 300) pour la recharge sans contact d'un dispositif de stockage d'énergie d'un implant (I) implanté dans le corps d'un être vivant, comprenant:

au moins une bobine autoportante (110, 210), s'étendant le long d'un axe de bobine et étant configurée pour générer un champ magnétique alternatif;
le corps est, lors d'une utilisation conforme à sa destination du chargeur (100, 200, 300), disposé spatialement par rapport à la bobine (110, 210) de telle sorte que le champ magnétique alternatif, qui s'étend dans la région située à l'intérieur de la bobine le long de l'axe de la bobine, pénètre dans le corps pour recharger le dispositif de stockage d'énergie.

**2.** Chargeur (100, 200, 300) selon la revendication 1, dans lequel le chargeur est configuré
pour faire tourner, au moins de manière spatialement bidimensionnelle, un vecteur du champ magnétique alternatif en vue de la recharge.

**3.** Chargeur (100, 200, 300) selon la revendication 1 ou 2, dans lequel le chargeur est configuré
pour faire tourner le vecteur du champ magnétique alternatif en vue de la recharge sans modification de l'amplitude correspondante.

**4.** Chargeur (100, 200, 300) selon la revendication 1, 2 ou 3, dans lequel
le corps est, lors d'une utilisation conforme à sa destination du chargeur, disposé par rapport à la bobine autoportante de telle sorte qu'un axe longitudinal du corps s'étende dans la direction de l'axe de la bobine et se trouve à l'intérieur de la bobine, le champ magnétique alternatif atteignant l'emplacement de l'implant.

**5.** Chargeur (100, 200, 300) selon la revendication 2, 3 ou 4, comprenant en outre:

un dispositif de suspension, qui maintient la bobine autoportante et est configuré pour faire pivoter la bobine par rapport au corps autour d'au moins un axe, de préférence autour de deux axes, afin de faire tourner le vecteur, dans lequel
le chargeur est configuré pour commander le dispositif de suspension afin de faire pivoter la bobine vers une orientation déterminée par rapport au corps en vue d'optimiser la recharge du dispositif de stockage d'énergie.

**6.** Chargeur (100, 200, 300) selon la revendication 5, dans lequel

le dispositif de suspension, qui maintient la bobine, est déplaçable par rapport au corps et/ou un dispositif de maintien du corps destiné à maintenir le corps est déplaçable par rapport à la bobine, et dans lequel

le chargeur est configuré pour déplacer le dispositif de suspension et/ou le dispositif de maintien du corps afin d'amener la bobine dans une position déterminée par rapport au corps en vue d'optimiser la recharge du dispositif de stockage d'énergie.

7. Chargeur (100, 200, 300) selon la revendication 5 ou 6, dans lequel

le chargeur comprend une unité de réception qui est configurée pour soit recevoir, soit interroger, un signal de qualité généré dans l'implant et reflétant le rendement de la recharge, et le chargeur est configuré pour, en fonction du signal de qualité, faire pivoter la bobine vers l'orientation déterminée et/ou amener la bobine dans la position déterminée afin d'optimiser la recharge.

8. Chargeur (100, 200, 300) selon l'une quelconque des revendications précédentes, dans lequel une densité de flux magnétique du champ magnétique alternatif le long de l'axe de la bobine a une valeur B, telle que :

$$1{,}0\,mT <= B <= 20{,}0\,mT,$$

de préférence

$$2{,}0\,mT <= B <= 20{,}0\,mT,$$

$$2{,}5\,mT <= B <= 8{,}0\,mT,$$

$$3{,}5\,mT <= B <= 7{,}0\,mT,$$

$$4{,}5\,mT <= B <= 6{,}0\,mT,$$

$$4{,}8\,mT <= B <= 5{,}2\,mT,$$

ou

$$5{,}0\,mT = B.$$

9. Chargeur (100, 200, 300) selon l'une des revendications 1, 2 ou 3, dans lequel la bobine est constituée de deux sous-bobines espacées l'une de l'autre d'une distance R1 le long d'un même axe, lesquelles coopèrent de telle sorte que le champ magnétique alternatif le long de l'axe de la bobine traverse respectivement les sous-bobines ainsi qu'une région située entre les sous-bobines; R1 étant de préférence égal à D/2 (bobine de Helmholtz), et le corps est, lors d'une utilisation conforme à sa destination du chargeur, disposé par rapport aux sous-bobines de telle sorte que le champ magnétique alternatif, de préférence essentiellement homogène, situé entre les sous-bobines, pénètre dans le corps pour la recharge du dispositif de stockage d'énergie.

10. Chargeur (100, 200, 300) selon la revendication 9, comprenant en outre:

un dispositif de suspension, qui maintient les deux sous-bobines de la première bobine et est configuré pour faire pivoter la première bobine par rapport au corps autour d'au moins un axe, de préférence autour de deux axes, afin de faire tourner le vecteur, et dans lequel le chargeur est configuré pour commander le dispositif de suspension afin de faire pivoter les deux sous-bobines de la première bobine vers une orientation déterminée par rapport au corps en vue d'optimiser la recharge du dispositif de stockage d'énergie.

11. Chargeur (100, 200, 300) selon la revendication 9, dans lequel le chargeur est configuré pour faire pivoter les deux sous-bobines de la première bobine, en fonction d'un signal de qualité émis par l'implant et reflétant le rendement de la recharge, vers l'orientation déterminée par rapport au corps en vue d'optimiser la recharge du dispositif de stockage d'énergie.

12. Chargeur (100, 200, 300) selon la revendication 8 ou 9, comprenant en outre:

une seconde bobine, s'étendant le long d'un axe de bobine et constituée de deux sous-bobines, lesquelles sont disposées l'une par rapport à l'autre à une distance R2 de telle sorte qu'une région se trouve entre les sous-bobines de la seconde bobine, dans laquelle les axes de bobine de la première bobine et de la seconde bobine s'étendent de manière transversale, de préférence perpendiculaire, l'un par rapport à l'autre, de telle sorte que les champs magnétiques situés entre les sous-bobines de la première bobine et de la seconde bobine se superposent dans une région commune, et le chargeur est configuré pour commander la première bobine et la seconde bobine, en vue d'optimiser la recharge du dispositif de stockage d'énergie, de telle sorte qu'une direction du vecteur du champ magnétique alternatif dans la région de superposition tourne de manière bidimensionnelle (dans un plan).

**13.** Chargeur (100, 200, 300) selon la revendication 12, comprenant en outre:

une troisième bobine, s'étendant le long d'un axe de bobine et constituée de deux sous-bobines, lesquelles sont disposées l'une par rapport à l'autre à une distance R3 de telle sorte qu'une région se trouve entre les sous-bobines de la troisième bobine, dans laquelle
les axes de bobine de la première bobine, de la seconde bobine et de la troisième bobine s'étendent de manière transversale les uns par rapport aux autres, de préférence le long des coordonnées spatiales X, Y et Z, de telle sorte que les champs magnétiques situés entre les sous-bobines de la première bobine, de la seconde bobine et de la troisième bobine se superposent dans la région commune, et
le chargeur est configuré pour commander la première bobine, la seconde bobine et la troisième bobine, en vue d'optimiser la recharge du dispositif de stockage d'énergie, de telle sorte que la direction du vecteur du champ magnétique alternatif dans la région de superposition tourne de manière tridimensionnelle.

**14.** Chargeur (100, 200, 300) selon la revendication 11 ou 12, dans lequel

le chargeur est configuré pour soit recevoir, soit interroger, un signal de qualité généré dans l'implant et reflétant le rendement de la recharge, et
le chargeur est configuré pour, en vue d'optimiser la recharge du dispositif de stockage d'énergie, amener le vecteur du champ magnétique alternatif dans la région de superposition à une position définie.

Fig. 1B

Fig. 1A

(x,y,z)=(0,0,0)

Fig. 1C

radiale magnetische Flussdichteverteilung in der X-Y-Ebene bei Z=0

Fig. 1D

Fig. 2A

Fig. 2B

EP 4 284 496 B1

**Fig. 3**

EP 4 284 496 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102018205940 A1
- WO 2009051539 A1
- WO 2017025606 A1
- US 2012146575 A1
- DE 102019124435
- EP 3756726 A2